# EUROPEAN PATENT SPECIFICATION

(11) **EP 4 173 647 B1**
(45) Date of publication and mention of the grant of the patent: **10.04.2024**
(21) Application number: 22204780.5
(22) Date of filing: 31.10.2022
(51) Int. Cl.: A61L 27/04, A61L 27/06, A61L 27/30, A61L 27/32, A61L 27/34

(54) **HYDROXYAPATITE BASED COMPOSITES AND FILMS THEREOF**
VERBUNDSTOFFE AUF HYDROXYAPATITBASIS UND FOLIEN DARAUS
COMPOSITES À BASE D'HYDROXYAPATITE ET FILMS ASSOCIÉS

(30) Priority: 01.11.2021 US 202117515890
(43) Date of publication of application: 03.05.2023
(73) Proprietor: Yeda Research and Development Co. Ltd., 7610002 Rehovot (IL); Holon Academic Institute of Technology, 5810201 Holon (IL)
(72) Inventor: TENNE, Reshef, 7623135 Rehovot (IL); SHALOM, Hila, 7610002 Rehovot (IL); RAPOPORT, Lev, 58678xx Holon (IL); GOLAN, Ofek, 7610002 Rehovot (IL)
(74) Representative: Pearl Cohen Zedek Latzer Baratz UK LLP

(56) References cited:
- WO-A1-2019/162943

## Description

### FIELD OF THE INVENTION

This invention is directed to composites and films comprising hydroxyapatite, biodegradable polymer, a biocompatible surfactant with inorganic fullerene-like (IF) nanoparticles or inorganic nanotubes (INT); methods of preparation and uses thereof.

### BACKGROUND OF THE INVENTION

Self-lubricating solid-state films are used for a variety of applications including the automotive, medical devices, power generation, machining, shipping, aerospace industries as well as many others. Often such films are a nanocomposite made of hard matrix containing a minority phase of a soft metal like copper or silver, or impregnated nanoparticles with good tribological performance [Basnyat, P.; et al. Mechanical and tribological properties of CrAlN-Ag self-lubricating films. Surf. Coat. Technol. 2007, 202, 1011-1016].

More recently, self-lubricating films containing **carbon nanotubes** [Moghadam, A.D.; et al. Mechanical and tribological properties of self-lubricating metal matrix nanocomposites reinforced by carbon nanotubes (CNTs) and graphene-A review. Compos. Part B 2015, 77, 402-420], **MoS₂** [Liu, E.Y.; Wang, W.Z.; Gao, Y.M.; Jia, J.H. Tribological properties of Ni-based self-lubricating composites with addition of silver and molybdenum disulfide. Tribol. Int. 2013, 57, 235-241.] and **WS₂** nanoparticles [Lian, Y.; et al. Friction and wear behavior of WS2/Zr self-lubricating soft coatings in dry sliding against 40Cr-hardened steel balls. Tribol. Lett. 2014, 53, 237-246] have been described.

Hydroxyapatite (Ca₁₀(PO₄)₆(OH))₂ is the major component of natural bone. Hydroxyapatite (HA) has many stoichiometric phases, called calcium phosphate phases, with their Ca/P ratio varying between 1.67 and 1.5 . Hydroxyapatite (HA, Ca₁₀(PO₄)₆(OH)₂) is used as a bone replacement material in a variety of orthopedic implants and artificial prostheses [Petit, R. The use of hydroxyapatite in orthopaedic surgery: A ten-year review. Eur. J. Orthop. Surg. Traumatol. 1999, 9, 71-74]. Given the fact that already 15% of the population is above 65 and increasing, artificial orthopedic implants have become a major health issue. However, this material suffers from high wear and poor fracture toughness and is very brittle.

To alleviate these problems various methods were conceived including incorporation of nanoparticles (NP) into the HA films. In particular, HA films containing **carbon** [Lahiri, D.; et al. Carbon nanotube toughened hydroxyapatite by spark plasma sintering: Microstructural evolution and multiscale tribological properties. Carbon 2010, 48, 3103-3120] and **boron nitride** nanotubes [Lahiri, D.; et al. Boron nitride nanotube reinforced hydroxyapatite composite: Mechanical and tribological performance and in-vitro biocompatibility to osteoblasts. J. Mech. Behav. Biomed. 2011, 4, 44-56] were prepared by spark plasma sintering technique.

Frequently, the HA phase also contains associated minerals and materials, including brushite and portlandite. Brushite-(CaH(PO₄)·2H₂O) is a metastable compound in physiological conditions and therefore it transforms into hydroxyapatite after implantation of a prostheses [Theiss, F.; Apelt, D.; Brand, B.; Kutter, A.; Zlinszky, K.; Bohner, M. Biocompatibility and resorption of a brushite calcium phosphate cement. Biomaterials 2005, 26, 4383-4394].

HA can be synthesized via a hydrothermal reaction of CaO and monetite (CaHPO₄). High concentration of calcium oxide in the reaction leads to the formation of excess portlandite-Ca(OH)₂, while low concentration of calcium oxide results in hydroxyapatite [Rodriguez-Lugo, V.; et al. Synthesis and structural characterization of hydroxyapatite obtained from CaO and CaHPO4 by a hydrothermal method. Mater. Res. Innov. 2005, 9, 20-22]. Biphasic calcium phosphate (BCP) is an intimate mixture of two phases of HA and β-TCP (Ca₃(PO₄)₂) in variety of ratios, which appears after annealing of HA above 700 °C [Kuo, M.C.; Yen, S.K. The process of electrochemical deposited hydroxyapatite coatings on biomedical titanium at room temperature. Mater. Sci. Eng. C 2002, 20, 153-160.].

Poly(L-lactic acid) (PLLA) is a biocompatible, degradable and semi-crystalline polymer. It is one of the most investigated polymers for biodegradable/biocompatible applications including food packaging, medical implants, tissue engineering scaffolds and many more. PLLA can be processed by various techniques, including extrusion, solvent casting, 3D printing, electrospinning, etc.

Nanoslabs (graphene-like) of MoS₂ and numerous other layered materials are currently studied intensively for variety of optoelectronic as well as for energy harvesting and energy-storage devices [Manzeli, S.; et al. 2D transition metal dichalcogenides. Nat. Rev. Mater. 2 2017, 44, 16399-16404]. WS₂ and MoS₂ nanoparticles with fullerene-like (IF) structure were found to perform well as solid lubricants [Rapoport, L.; et al. Hollow nanoparticles of WS2 as potential solid-state lubricants. Nature 1997, 387, 791-793; (ii) Rosentsveig, R.; et al. Fullerene-like MoS2 nanoparticles and their tribological behavior. Tribol. Lett. 2009, 36, 175-182]. They are presently used in various commercial products, mostly as additives to lubricating fluids, greases, metal working fluids and in high performance bearings.

Recently, doping of IF-MoSz nanoparticles with minute amounts (<200 ppm) of rhenium atoms (Re:IF-MoS₂) was demonstrated [ Yadgarov, L.; et al. Tribological studies of rhenium doped fullerene-like MoS2 nanoparticles in boundary, mixed and elasto-hydrodynamic lubrication conditions. Wear 2013, 297, 1103-1110].

One of the most critical aspects of the usage of nanomaterials is their toxicity and biocompatibility. In contrast to various other nanoparticles, the IF NP were found to be non-toxic in general, up to a high dosage (>20 µg/mL). These findings are beneficial for the development of medical technologies based on such nanoparticles.

In the present invention, HA based films are impregnated with doped inorganic fullerene-like (IF) nanoparticles (such as: Re:IF-WS₂, Nb: IF-WS₂, Re:IF-MoS₂, Nb:IF-MoS₂) or with doped inorganic nanotubes (INT) (such as: Re:INT-WS₂, Nb: NT-WS₂, Re:INT-MoS₂, Nb:INT-MoS₂) leading to substantial improvement in their tribological behavior.

### SUMMARY OF THE INVENTION

The present invention provides a composite comprising a hydrophobic biodegradable polymer, hydroxyapatite [Ca₁₀(PO₄)₆(OH)₂)] nanoparticles, a biocompatible surfactant and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons. In some embodiments, the biodegradable polymer is poly(lactic acid) (PLA), Poly(L-lactide) (PLLA) or poly-D-lactide (PDLA). In some embodiments, the biocompatible surfactant is oleic acid (C₁₈H₃₄O₂).

In some embodiments, the composite comprises PLLA (Poly(L-lactic acid), hydroxyapatite [Ca₁₀(PO₄)₆(OH)₂)] nanoparticles, oleic acid, and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; and the chalcogenide is selected from S, Se, Te.

In some embodiments, the composite described herein is deposited on a substrate forming a film. In other embodiments, the film is formed by solvent casting. In other embodiments, the film described herein exhibits improved mechanical and thermal properties compared to the films prepared from the PLLA and HA alone, which is advantageous for medical applications. In some other applications the film is used for extruding filaments.

### BRIEF DESCRIPTION OF THE DRAWINGS

The scope of protection is defined by the claims. The invention, however, both as to organization and method of operation, together with objects, features, and advantages thereof, may best be understood by reference to the following detailed description when read with the accompanying drawings in which:

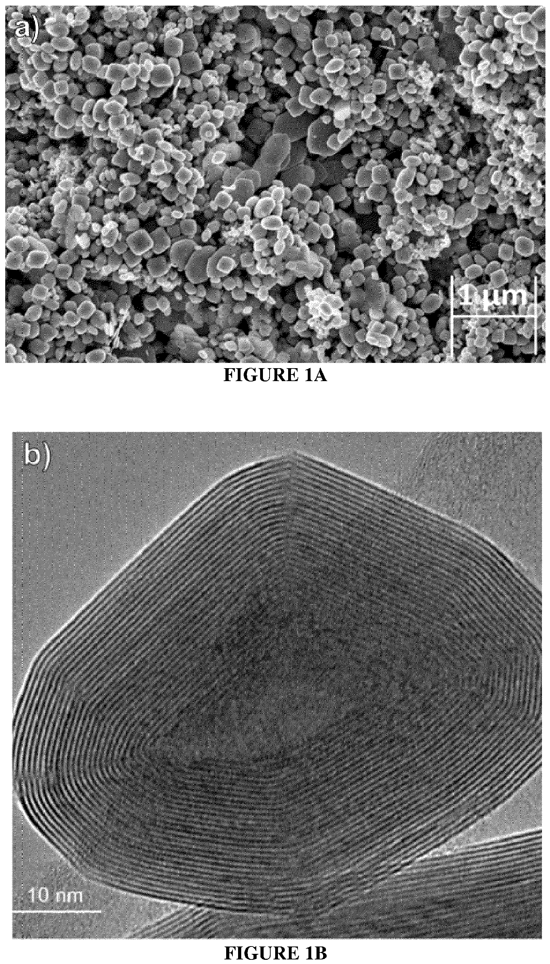

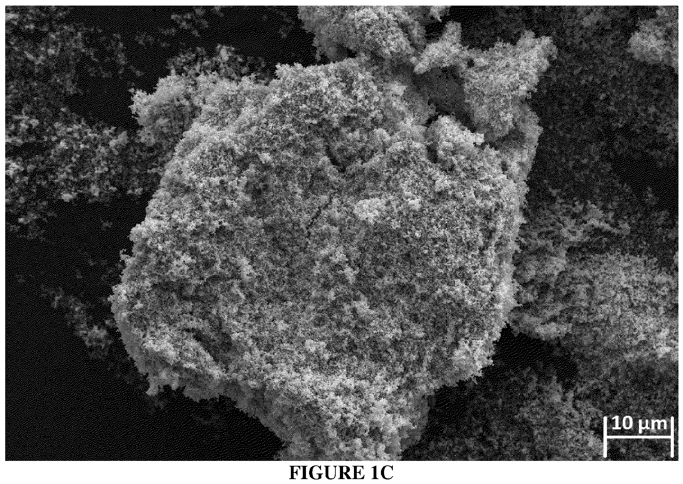

**Figures 1A-1C** present SEM images of Re: IF-MoSz nanoparticles. **Figure 1A****:** shows high-resolution scanning electron microscope (HRSEM) micrograph of the Re-doped IF NP powder in In-lens detector 5kV. The oblate shape of the nanoparticles with smooth surfaces is clearly delineated. The size range of the nanoparticles is 70-170 nm with a minor content (<10%) of NP larger than 200 nm. **Figure 1B** shows high-resolution transmission electron microscopy (HRTEM) image of one such nanoparticle made of some 20 closed and nested layers of MoSz. The crystalline perfection and atomically smooth (sulfur-terminated) surface of the IF NP contributes to their excellent mechanical and tribological performance. **Figure 1C** shows SEM view of an agglomerate of Re:IF-MoS₂ nanoparticles. The synthesized nanoparticles are highly agglomerated and must be deagglomerated before use. Light sonication suffices to disperse them well in aqueous or ethanolic suspensions.
**Figures 2A-2B** present HRSEM pictures of HA with Re:IF-MoS₂ nanoparticles coating obtained from ***solution A*** on porous titanium substrate in two magnifications: **(****Figure 2A****)** 100 µm; **(****Figure 2B****)** 2 µm. The film is continuous but visibly is heavily cracked.
**Figures 3A-3D** present HRSEM images of the HA film with Re:IF-MoS₂ obtained from ***solution A*** after 2 hours **(****Figure 3A****),** 3 hours **(****Figure 3B****),** and 4 hours **(****Figure 3C****)** deposition. The Re:IF-MoS₂ nanoparticles in the film **(****Figure 3C****)** are observed in the backscattering electron (BSE) mode **(****Figure 3D****).** The arrows in **Figure 3D** point on the Re:IF-MoS₂ nanoparticles occluded in the HA film.
**Figures 4A-4B** present zeta-potential vs. pH for Re:IF-MoS₂ nanoparticles. Figure 4A presents zeta-potential vs pH for Re:IF-MoS₂ nanoparticles in ***solutions A, B and C.*** The (positive) zeta-potential of the solutions used for EPD of the HA + IF film are marked by enlarged symbols. **Figure 4B** presents zeta-potential vs pH for Re:IF-MoS₂ nanoparticles in different solutions.

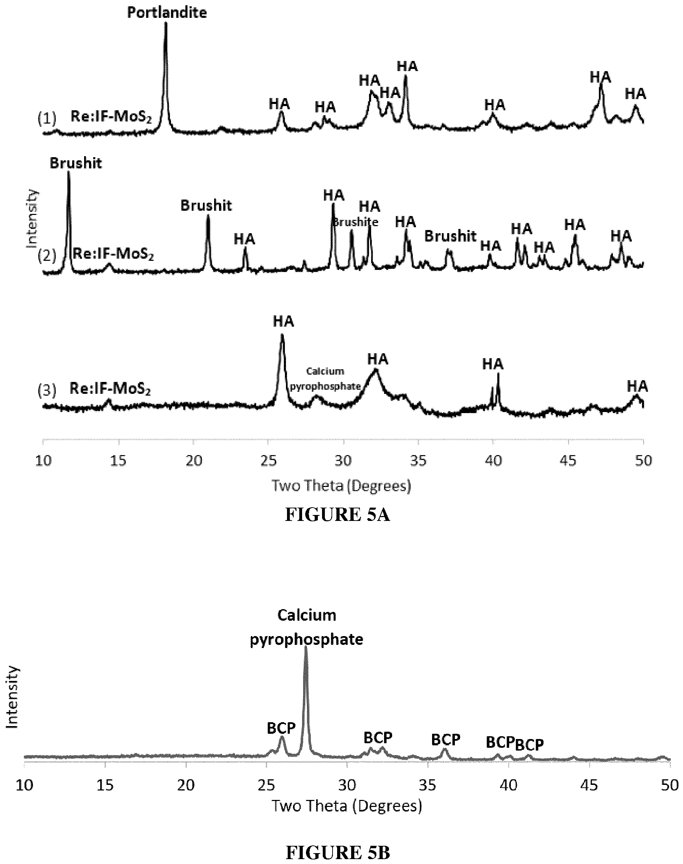

**Figures 5A-5B** present XRD patterns of the HA films incorporating Re:IF-MoS₂ nanoparticles. **Figure 5A** presents the XRD patterns of the different coatings obtained from ***solution* A** (1), ***solution B*** (2) and ***solution* C** (3). **Figure 5B** presents XRD pattern of the film obtained from ***solution A*** (3 h) after annealing (700 °C for 1 h). Here, a strong crystalline peak associated with calcium pyrophosphate phase (Ca₂(P₂O₇)) is observed. This phase is obtained through water evaporation from the HA (Ca₁₀(PO₄)₆(OH)₂) film. The presence of the Re:IF-MoS₂ nanoparticles did not change appreciably upon annealing, suggesting that these NPs are thermally stable at the annealing conditions.
**Figure 6** presents XRD patterns of films obtained from ***solution A*** without the Re:IF-MoSz NP (a) and (with the IF NP) for different deposition periods: after 2 hours (b), 3 hours (c) and 4 hours (d).
**Figure 7** presents Raman spectra of HA powder film without (a) and with the Re:IF-MoS₂ nanoparticles obtained from ***solution A*** for different EPD periods: after 2 hours (b), 3 hours (c) and 4 hours (d).
**Figures 8A-8D** present optical images of wear on the ball and inside the track of HA film without **(****Figure 8A****)** and with the Re:IF-MoS₂ nanoparticles obtained from ***solution A*** for different periods: after 2 hours **(****Figure 8B****),** 3 hours **(****Figure 8C****)** and 4 hours **(****Figure 8D****)** on anodized titanium.
**Figures 9A-9B** present SEM images of HA with Re:IF-MoS₂ nanoparticles coating obtained from ***solution B*** (3 h deposition time) on porous titanium substrate in different magnifications.
**Figures 10A-10B** present SEM pictures of HA with Re:IF-MoS₂ nanoparticles coating obtained from ***solution C*** (1 h deposition time) on porous titanium substrate in different magnifications.
**Figures 11A-11D** SEM images of titanium surface **(a,b)** before and **(c,d)** after surface treatment in different magnifications.
**Figures 12A-12C** SEM images of porous titanium after anodization in different magnifications, the average diameter of the pores (tubes) is 100 nm.
**Figure 13** depicts XRD patterns of PLLA film and HA/INT-WS₂/PLLA nanocomposites.
**Figures 14A-14C** depict HR-SEM images (secondary electrons- SE mode) of pure HA powder **(****Figure 14A****)** and PLLA film reinforced with 40 wt% HA observed in the SE mode in two magnifications (scale bars) 2 µm, the arrows point to the spherical agglomerates of HA nanoparticles. **(****Figure 14B****)** and 1 µm, the arrows point to pits formed by pull-out of the HA agglomerates during breaking of the film **(****Figure 14C****).**
**Figures 15A-15B** depict HR-SEM images of PLLA film reinforced with 40 wt% HA and 0.75 wt% INT-WS₂ film in SE mode under 1 µm magnifications (scale bar) **(****Figure 15A****)** and BSE mode in 500 nm **(****Figure 15B****).**
**Figures 16A-16D** depict HR-SEM image of PLLA film reinforced with 40 wt% HA and 0.75 wt% INT-WS₂ in SE mode **(****Figure 16A****)** and EDS elemental mapping of the same area: carbon **(****Figure 16B****),** phosphorus **(****Figure 16C****)** and tungsten **(****Figure 16D****).**
**Figure 17** depicts stress-strain curves of PLLA film and its HA/INT-WS₂/PLLA nanocomposites.
**Figure 18** depicts Micro Vickers hardness test of PLLA film and HA/INT-WS₂/PLLA nanocomposites.
**Figures 19A-19B** depict DSC thermograms of heating **(****Figure 19A****)** and cooling **(****Figure 19B****)** of PLLA film and HA/INT-WS₂/PLLA nanocomposites.
**Figure 20** depicts Raman spectra of the different PLLA films and PLLA/HA/INT-WS₂ nanocomposites films. The Raman peak of the oleic acid is shown in the inset.
**Figure 21** depicts FTIR spectra of different PLLA/HA/INT-WS₂ nanocomposite films.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The present invention is directed to a composite comprising a hydrophobic biodegradable polymer, hydroxyapatite [Ca₁₀(PO₄)₆(OH)₂)] nanoparticles, a biocompatible surfactant and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons.

In some embodiments, the composite is deposited on a substrate to form a film..

In other embodiments, the biodegradable polymer is poly(lactic acid) (PLA), Poly(L-lactide) (PLLA) or poly-D-lactide (PDLA).

Examples of fatty acids include palmitic, stearic, arachidic, behenic, palmitoleic, sapienic, elaidic, oleic, vaccenic, linoleic, linoelaidic, a-linolenic, arachidonic, eicosapentaenoic, erucic, docosahexaenoic acid or any combination thereof. In other embodiments, the biocompatible surfactant is oleic acid (C₁₈H₃₄O₂).

In some embodiments, the composite comprises PLLA (Poly(L-lactic acid), hydroxyapatite [Ca₁₀(PO₄)₆(OH)₂)] nanoparticles, oleic acid, and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; and the chalcogenide is selected from S, Se, Te.

Incorporated hydroxyapatite in the PLLA matrix improves the flexibility of the bioceramic HA and consequently, produce biodegradable ceramic-polymer composites, which is an alternative to the traditional materials used for implants or bone repair and for tissue engineering. However, both PLLA and HA and their composites suffer from low toughness, which limit their application in the human body. Reinforcing the PLLA/HA composite with inorganic fullerene-like nanoparticles (IF-NPs) or inorganic nanotubes (INT) (such as INT-WS₂) can remedy this disadvantage. The INT-WS₂ are multiwall nanostructures 1-20 µm long with diameter of 30-150 nm (aspect ratio of 50-100 and even larger). They are nontoxic with very good mechanical properties (Young's modulus 150-170 GPa, bending modulus of 217 GPa, tensile strength between 10-22 GPa, and strain ε > 10%).

HA does not disperse well in the PLLA matrix and tends to agglomerate as secondary particles a few micrometers in size. This is because HA is hydrophilic, while the organic solvents used to dissolve the polymers are mostly hydrophobic. However, a biocompatible surfactant such as oleic acid (OA), which is an amphiphilic surfactant, may be used to mediate the interaction between the HA (hydrophilic ceramic) and the hydrophobic polymer, like PLLA. Therefore, the fatty acid (e.g. oleic acid) induces a homogeneous dispersion of the HA in the hydrophobic polymer (e.g. PLLA) matrix; and eliminates phase separation or any additional agglomeration within the matrix.

In some embodiments, this invention is directed to a film comprising the composition/composite of this invention.

In some embodiments, this invention is directed to a film comprising a hydrophobic biodegradable polymer, hydroxyapatite [Ca₁₀(PO₄)₆(OH)₂)] nanoparticles, a biocompatible surfactant, and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons.

In some embodiments, this invention is directed to a film comprising PLLA (Poly(L-lactic acid), hydroxyapatite [Ca₁₀(PO₄)₆(OH)₂)] nanoparticles, oleic acid, and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; and the chalcogenide is selected from S, Se, Te.

In other embodiments, the inorganic nanotubes (INT) are WS₂.

In other embodiments, the inorganic fullerene-like nanoparticles (IF-NPs) or the inorganic nanotubes (INT) are doped by rhenium and niobium.

Inorganic Fullerene-like (IF) nanoparticles and/or inorganic nanotubes (INT) of this invention each having the formula A₁₋ₓBₓ-chalcognide wherein A is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and B (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; and x is below or equal 0.003, and the chalcogenide is selected from S, Se, Te. In other embodiments, x is below or equal 0.001.

For example, doped IF-NP or doped INT of the invention may be IF-Mo₁₋ₓNbₓS₂, IF-Mo₁₋ₓReₓS₂, INT-Mo₁₋ₓNbₓS₂, INT-Mo₁₋ₓReₓS₂, IF-W₁₋ₓNbₓS₂, IF-W₁₋ₓReₓS₂, INT-W₁₋ₓNbₓS₂, INT-W₁₋xRexS₂ or the alloys of WMoSz, WMoSe₂, TiWSz, TiWSe₂, where Nb or Re are doped therein. Within the alloys of the invention, taking WMo, TiW for example, the ratio between W and Mo or Ti and W may be 0.65-0.75 of one metal or transition metal and 0.25-0.35 of the other metal or transition metal, e.g. W_{0.7}Mo_{0.29}Nb_{0.01}S₂ (given with the percentage of the Nb dopant).

In one embodiment, the rhenium or niobium atoms serve as dopants in the lattice of the IF-NPs/INTs. The dopants substitute for the molybdenum or tungsten atoms, which lead to an excess of negative charge carriers being trapped on the IF-NPs/INT surfaces.

In other embodiments, the concentration of the dopants is below or equal to 0.3 wt%. In other embodiments, the concentration of the dopants is between 0.01 to 0.1 wt%. In other embodiments, the concentration of the dopants is between 0.01 to 0.07 wt%. In other embodiments, the concentration of the dopants is between 0.01 to 0.05 wt%.

The doped IF-nanoparticles/inorganic nanotubes behave like charged colloids, which do not agglomerate and form stable suspensions in oils and various fluids. This is in contrast to the undoped IF-NPs/INTs, as their structure allows rolling. Additionally, the doped IF-NPs and doped INTs have higher conductivity, higher carrier density, lower activation energy, and lower resistance than the undoped ones.

In some embodiments, the composition of the film further comprises brushite, portlandite, other HA minerals or combination thereof.

In some embodiments, this invention is directed to a composition/composite and/or a film comprising PLLA (Poly(L-lactic acid), hydroxyapatite [Ca₁₀(PO₄)₆(OH)₂)] nanoparticles, oleic acid and inorganic fullerene-like nanoparticles or inorganic nanotubes; wherein the inorganic fullerene-like nanoparticles or inorganic nanotubes is A₁₋ₓBₓ-chalcogenide where A is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and B (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; and the chalcogenide is selected from S, Se, Te; wherein the concentration of inorganic fullerene-like nanoparticles or inorganic nanotubes is between 0.1 wt% to 5 wt% of the composition. In other embodiments, the concentration of HA is between 20 wt% to 60 wt% of the composition.

In some embodiments, this invention is directed to a composite and/or a film comprising a hydrophobic biodegradable polymer, a biocompatible surfactant, hydroxyapatite [Ca₁₀(PO₄)₆(OH)₂)] nanoparticles, and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons; wherein the concentration of inorganic fullerene-like nanoparticles or inorganic nanotubes is between 0.1 wt% to 5 wt% of the composition. In another embodiment between 0.1 wt% to 1 wt%. In another embodiment between 0.2 wt% to 1 wt%. In another embodiment between 0.1 wt% to 2 wt%. In another embodiment between 0.1 wt% to 3 wt%. In another embodiment between 0.5 wt% to 3 wt%. In another embodiment between 0.5 wt% to 2 wt%. In another embodiment between 1 wt% to 5 wt%. In another embodiment, the biodegradable polymer is PLLA. In another embodiment, the biocompatible surfactant is oleic acid.

In some embodiments, this invention is directed to a composition and/or a film comprising a hydrophobic biodegradable polymer, a biocompatible surfactant, hydroxyapatite [Ca₁₀(PO₄)₆(OH)₂)] nanoparticles, and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons; wherein the concentration of HA is between 20 wt% to 60 wt% of the composition. In another embodiment between 20 wt% to 30 wt%. In another embodiment between 20 wt% to 40 wt%. In another embodiment between 20 wt% to 50 wt%. In another embodiment between 30 wt% to 40 wt%. In another embodiment between 30 wt% to 50 wt%. In another embodiment between 30 wt% to 60 wt%. In another embodiment, the biodegradable polymer is PLLA. In another embodiment, the biocompatible surfactant is oleic acid.

The term "composite" or "composition" is used herein interchangeably referring to hydroxyapatite is embedded in different matrices (biodegradable polymers), such as the PLLA. The composite/composition of this invention further comprises inorganic particles selected from inorganic fullerene-like nanoparticles or inorganic nanotubes and a biocompatible surfactant (i.e. oleic acid)

In some embodiments, this invention is directed to a film comprising PLLA (Poly(L-lactic acid), hydroxyapatite [Ca₁₀(PO₄)₆(OH)₂)] nanoparticles, oleic acid and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; and the chalcogenide is selected from S, Se, Te; wherein the film is prepared by solvent casting on a solid surface, wherein the film HA nanoparticles and the organic fullerene-like nanoparticles or inorganic nanotubes within the film are dispersed in the PLLA.

The present invention also provides a method of coating of a substrate by a film, wherein the film comprises the composite described herein, wherein the method comprises solvent casting.

In other embodiment the film provides Young's modulus being 1.5 to 3 times higher compared to a film comprising PLLA and HA. In other embodiments, the film provides a toughness being 2 to 10 times higher compared to a film comprising PLLA and HA. In other embodiments, the film provides a hardness being 1.5 to 3 times higher compared to PLLA film. In other embodiment, the film provides higher thermal stability compared to a PLLA film or a film comprising PLLA and HA. In other embodiment, there is no chemical bonding between each of the PLLA, HA and the inorganic fullerene-like nanoparticles or inorganic nanotubes.

In some embodiments the film provides an improved hardness by 1.2 to 3, Young Modules by 1.5 to 3, Toughness by 2 to 5, Yield Strength by 1.2 to 3 and/or Strain at failure by 1.1 to 3 compared to PLLA film.

In other embodiments, the film is formed by mixing each of PPLA, HA and INT or IF in an organic solvent followed solvent casting on a solid substrate and drying the formed film. In another embodiment, the organic solvent is chloroform, THF or mixtures thereof. In other embodiment, the oleic acid is used to homogenize the mixture between the HA phase and the other two components (PLLA and INT/IF). In other embodiments, minute amounts of a solvent remain in the film. In other embodiments, no solvent remains in the film. In other embodiments, other means of film formation include hot-pressing, or extrusion and subsequent 3D printing.

The control of the interfacial interaction between the two majority phases, i.e. PLLA and HA and the minority phase- INT-WS₂ has major implications on the mechanical stability of the nanocomposite subdued to different stress and environmental conditions. The oleic acid, which is used for compatibilization of HA in different polymer phases, was found to be indispensable. First, both oleic acid and HA nanoparticles are non-toxic and biocompatible phases. The FTIR, XRD and Raman measurements as presented in Example 6 do not reveal any specific chemical bonding between each of the four components (PLLA, HA, OA and INT) or a new phase forming during the preparation of the film.

It remains to be seen if other specific functionalization processes of the nanotubes surface could further improve their interfacial interaction with the matrix and influence the mechanical behavior of the nanocomposite without sacrificing its biocompatibility. The INT and IF within the compositions described herein have no specific interaction with the polymer-HA, their large surface area and aspect ratio (50-100) as well as their mechanical strength (10-22 GPa), large strain (10%), non-toxic nature and their facile dispersion make the INT/IF suited for reinforcing biodegradable polymer, even if added in minute amounts (-0.1 wt%~ 5 wt%).

In some embodiments, this application discloses a method for preparing the composites as described hereinabove. In one embodiment, this application discloses a method for preparing a composite comprising a biodegradable polymer, hydroxyapatite [Ca₁₀(PO₄)₆(OH)₂)] nanoparticles, a biocompatible surfactant and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons, wherein the method comprises:
- dispersing the inorganic fullerene-like nanoparticles or inorganic nanotubes in a first solvent;
- annealing the inorganic fullerene-like nanoparticles or inorganic nanotubes;
- dissolving the biodegradable polymer in a second solvent;
- dispersing the hydroxyapatite nanoparticles in a mixture of a third solvent with the biocompatible surfactant;
- dispersing the vacuum annealed inorganic fullerene-like nanoparticles or inorganic nanotubes in a fourth solvent;
- mixing the dissolved biodegradable polymer solution with the dispersed hydroxyapatite nanoparticles solution and the dispersed inorganic fullerene-like nanoparticles or inorganic nanotubes solution;
- transferring the obtained mixture to a plate;
- drying the transferred mixture; and
- annealing the dried mixture to afford the composite.

In some embodiments, this invention provides a composite comprising a biodegradable polymer, hydroxyapatite [Ca₁₀(PO₄)₆(OH)₂)] nanoparticles, a biocompatible surfactant and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons, wherein the composite is prepared by the following method which comprises the following:
- dispersing the inorganic fullerene-like nanoparticles or inorganic nanotubes in a first solvent;
- annealing the dispersed inorganic fullerene-like nanoparticles or inorganic nanotubes;
- dissolving the biodegradable polymer in a second solvent;
- dispersing the hydroxyapatite nanoparticles in a mixture of a third solvent with the biocompatible surfactant;
- dispersing the vacuum annealed inorganic fullerene-like nanoparticles or inorganic nanotubes in a fourth solvent;
- mixing the dissolved biodegradable polymer solution with the dispersed hydroxyapatite nanoparticles solution and the dispersed inorganic fullerene-like nanoparticles or inorganic nanotubes solution;
- transferring the obtained mixture to a plate;
- drying the transferred mixture; and
- annealing the dried mixture to afford the composite.

In other embodiments, the first, second, third and fourth solvent are each independently selected from ethanol, water, water-ethanol mixtures, dichloromethane (DCM), chloroform, diethyl ether, acetone, hexane, ethyl acetate, water, tetrahydrofuran (THF), acetonitrile, sulfolane, *N,N-*dimethylformamide (DMF), dimethylacetamide (DMA), dimethyl sulfoxide (DMSO), dioxane, ethanol, methanol or any combination thereof. In other embodiment, the first solvent is ethanol. In other embodiment, the second solvent is chloroform. In other embodiment, the third solvent is chloroform. In other embodiment, the fourth solvent is chloroform.

In other embodiments, the drying is performed in a hood (usually at room temperature). In some other embodiments, the drying is done under vacuum and/or heating, to a temperature of 30-160°C, 30-40°C, 40-50°C, 50-60°C, 60-70°C, 70-80°C, 80-90°C, 90-100°C, 100-110°C, 110-120°C, 120-130°C, 130-140°C, 140-150°C or 150-160°C. In other embodiments, the drying is performed for 1 hour - 10 days, 1-2 hours, 2-5 hours, 5-10 hours, 10-24 hours, 24 hours - 2 days, 2-5 days, or 5-10 days.

In other embodiments, the annealing is performed with or without vacuum. In some other embodiments, the annealing is done in a temperature of 30-160°C, 30-40°C, 40-50°C, 50-60°C, 60-70°C, 70-80°C, 80-90°C, 90-100°C, 100-110°C, 110-120°C, 120-130°C, 130-140°C, 140-150°C or 150-160°C. In other embodiments, the annealing is performed for 1 hour - 10 days, 1-2 hours, 2-5 hours, 5-10 hours, 10-24 hours, 24 hours - 2 days, 2-5 days, or 5-10 days. In another embodiment, the annealing is a vacuum annealing performed at 80°C for 1.5 hours or 5 days at 45°C.

In other embodiments, the plate is a Teflon, stainless steel, silicon or polypropylene plate; or any other plate as accepted in the art.

In some embodiment, the film of this invention is coated on a solid substrate. In other embodiments, the film described herein is formed by solvent casting. In other embodiments, the solid substrate is biocompatible. In other embodiments, the solid substrate is metallic biocompatible. In other embodiments the solid and biocompatible substrate is titanium, alloys of titanium, Ti₆Al₄V, Co-Cr alloys, magnesium, stainless steel, shape memory alloys of nickel-titanium, silver, tantalum, zirconium, novel ceramics such as alumina or zirconia or any other electrical-conductive substrate.

In other embodiment, the titanium is porous.

In other embodiment, to improve the coating of the film to the solid substrate the composition and/or film further comprises a polymeric binder. An example of a polymer binder includes a poly(lactic acid) (PLAs) based polymer.

Methods for coating a solid substrate with the composition of this invention to form a film on a solid substrate include; **(i)** electrophoretic deposition (solution); **(ii)** plasma spray (in vacuum); **(iii)** ion beam coating (in vacuum); **(iv)** e-beam evaporation [Cen Chen et al. Biomimetic apatite formation on calcium phosphate-coated titanium in Dulbecco's phosphate-buffered saline solution containing CaC12 with and without fibronectin, Acta Biomaterialia, (2010) 6, 2274-2281]; **(v)** thermal deposition; vacuum deposition [D. Predoi et al. Characteristics of hydroxyapatite thin films, J. Optoelect and Adv. Mat., (2007), 9(12), 3827-3831]; **(vi)** physical vapor deposition (PVD) [Ohad Goldbart et al. New Deposition Technique for Metal Films Containing Inorganic Fullerene-Like (IF) Nanoparticles, Chem Phys Chem, (2013), 14, 2125-2131; Olga Elianov MSc thesis submitted to the Faculty of Dental Medicine, Hadassah-Hebrew University, Jerusalem 91120, Israel (March 2018); **(vii)** aerosol deposition [C. Piccirillo, et al. Aerosol assisted chemical vapour deposition of hydroxyapatite-embedded titanium dioxide composite thin films, J. of photochem. And photobiol. A: Chemistry (2017), 332, 45-53]; **(viii)** sol gel deposition **(ix)** dip coating; or (x) solvent casting.

### Electrophoretic deposition:

The electrophoresis coating technique is an inexpensive process capable of a high deposition rate while maintaining control of the coating thickness and morphology on the metal. In addition, this technique has a wide range of materials permitting coating of variety of shapes and sizes, all resulting in a quality surface with uniform thickness. The electrophoresis coating technique also has high material efficiency and can be performed at low temperatures. The electrophoresis coating technique requires several steps, including surface treatments, which are used to clean the electrode from contaminants, improve the mechanical properties to create a uniform coating, and achieve better adhesion deposition. Electrophoresis coating is performed by dipping two electrodes into a container of electrolyte solution. A constant power supply creates an electrical field in the solution, which moves the charging colloid toward the opposite electrode. The deposition is obtained by chemical oxidation and reduction. The final step is an annealing process, and is done to achieve a smooth and continuous coating characterized by good adhesion to the surface. The electrophoretic deposition for coating the composition of this invention on a porous solid substrate is conducted at a relatively low temperature using an aqueous electrolyte containing calcium and phosphate salts. In this method, the calcium phosphate is deposited on the cathode as a result of a pH increase in the vicinity of the cathode and by the reduction of the H⁺ ion accompanying the generation of Hz gas and OH. ions. The production of Hz on the cathode's surface inhibits the nucleation or absorption of calcium phosphate on the cathode. Adding an alcohol such as ethanol to the electrolyte solution resolves this problem.

A metal substrate may be coated with a composition of this invention, wherein the coating method comprises electrophoretic deposition having an electrolyte comprising a calcium salt, a phosphate salt and doped inorganic fullerene like nanoparticles, and thereby forming a film of desirable composition on the substrate.

The coating process of the film depends on achieving the proper pH solution that allows quality coatings, which in turn, relies on the nanoparticles' zeta
potential measurement. In other embodiment, the composition has a positive zeta potential at pH below 6.5. In neutral pH (7) the nanoparticles are negatively charged,
which reflects the extra negative charge induced by native defects in the lattice and
chemisorbed negatively charged moieties, like OH- groups. This extra negative charge is neutralized in very low pH (up to pH=2) by positively charged chemical moieties, like protons, etc. In either very low or very high pH, the Debye screening radius is very small (few nm) leading to agglomeration of the nanoparticles and their precipitation. Thus, the electrophoresis coating process of the composition of this invention is performed at pH 6-7 to: 1) avoid damaging the surface of the nanoparticles; 2) provide a stable working solution; and 3) achieve a uniform coating of the substrate. Within this pH range, the nanoparticles gained a negative charge and the deposition was performed on the anode.

Coating a metal substrate with the composition of this invention may be performed by electrophoretic deposition. The metal substrate may be pretreated for example with carbon paper to obtain a smooth surface and then the metal substrate is anodized prior to the electrophoretic deposition. The metal substrate may be anodized in electrolyte solution containing a fluoride ion. The elecrophoretic deposition may be conducted as presented in Example 1.

Anodization is an electrochemical method for producing a protective layer on metal by forming a metal oxide layer which makes the metal substrate biocompatible. The metal oxide layer is a few tens of microns thick with micro pores to maintain homogeneity. The anodization process creates a porous surface, which improves and increases osteointegration (the functional connection between the human bone and the implant), and thereby increase the osteoblast adhesion (bone cell).

The electrophoretic deposition (EPD) may be conducted for between 2 to 5 hours, for example, conducted for 2, 3, 4 or 5 hours.

HA coatings containing up to 5 wt % doped IF-NPs or doped INTs may be deposited on a porous metallic biocompatible substrate by electrophoretic deposition using DC bias. The major phase in each coating is hydroxyapatite which incorporates small amounts of doped IF-NPs or doped INTs. The metal substrate may be a titanium substrate. In other embodiments, the doped inorganic fullerene-like nanoparticle is Re: IF-MoSz. In other embodiments, the doped inorganic fullerene-like nanoparticle is Re: IF-WS₂. In other embodiments, the doped inorganic fullerene-like nanoparticle is Nb: IF-MoSz. In other embodiments, the doped inorganic fullerene-like nanoparticle is Nb: IF-WS₂. In other embodiments, the doped inorganic nanotube is Re: INT-MoS₂. In other embodiments, the doped inorganic nanotube is Re: INT-WS₂. In other embodiments, the doped inorganic nanotube is Nb: INT-MoSz. In other embodiments, the doped inorganic nanotube is Nb: INT-WS₂.

In some embodiments, the film formed on the metal substrate has low friction coefficient of between 0.05 to 0.15. In another embodiment, the film formed by EPD on the metal substrate has low friction coefficient of between 0.05 to 0.1. In another embodiment, the low friction is maintained after annealing. In another embodiment, the film maintains its mechanical robustness.

### Uses thereof

Artificial bone implants became a major health concern. Hydroxyapatite (Ca₁₀(PO₄)₆(OH)₂; HA) is the main constituency of the bone. Hydroxyapatite is chemically similar to the calcium phosphate mineral present in bone and biological hard tissue.

The composition/composite and/or film of this invention are bioceramic suitable for implants and bone repair, and for tissue engineering.

In some embodiments, this invention provides an implant, a repaired bone or an engineered tissue comprising the composition/composite and/or film as described herein.

The composition/composite and film of this invention may be for use in dental and orthopedic implants having very low friction, good adhesion to the underlying rough substrate even under very high load (600 MPa). The composition and film of this invention have high biocompatibility, specifically as a bone substitute. The composition/film prepared according to this invention form a homogeneous structure, having slow degradability rate and both osteointegration and osteoconductive characteristics, which improve bone growth.

In some embodiments, this invention provides a dental or orthopedic implant comprising the composition of this invention. In other embodiments, this invention provides a dental or orthopedic implant comprising a film on a biocompatible substrate, wherein the film comprises the composition of this invention.

In some embodiments, an artificial bone implant comprising the composition of this invention may be used in bone regeneration therapy.

In some embodiments, an artificial bone implant comprising the composition of this invention may be for use in a method of osseointegration in a bone that needs to be improved. The artificial bone implant may comprise a biocompatible substrate coated by a film, wherein the film comprises the composition of this invention.

The advantages of this invention for osseointegration or for bone regeneration provide fast fixation and spontaneous binding of the HA to neighboring bone, having osteoconductive properties, resulting in deposition of biological apatite on the surface of the implant and thereby bone healing around the implant.

The following examples are presented in order to more fully illustrate the preferred embodiments of the invention.

### EXAMPLES

### EXAMPLE 1 (not according to the claimed invention)

### Preparation of a Film of Hydroxyapatite (HA) and rhenium doped fullerene like MoS₂ (Re:IF-MoS₂) on Titanium Substrate

A titanium electrode (30 × 5 × 0.3 mm, 97 wt% purity) was polished with silicon carbide paper to a mirror finish. It was subsequently cleaned by sonicating in a series of solvents, i.e., acetone, ethanol, methanol, isopropanol and finally distilled water, then dried under a nitrogen stream.

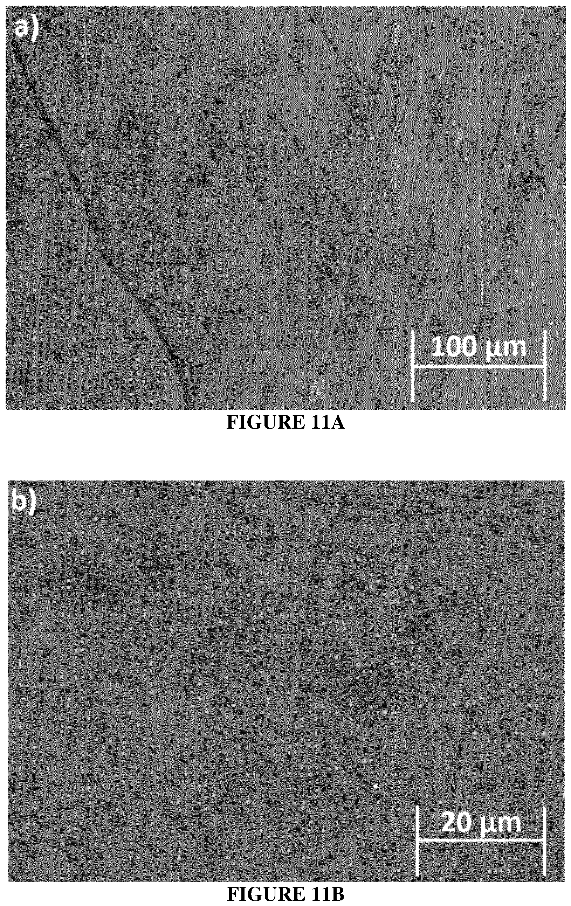

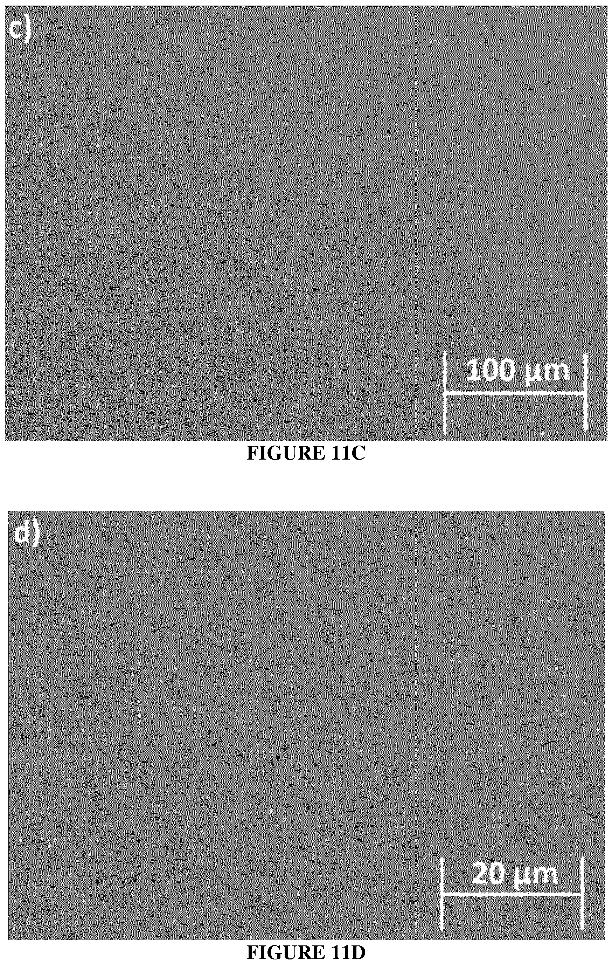

The surface morphology of the titanium before the pretreatment preceding the anodization is presented in Figures 11A-11B. Visibly, the fresh surface was heavily contaminated with a dense network of scratches. After treatment of the titanium with different solvents, a smooth surface with low density of scratches and clean from contaminants was obtained (Figures 11C-1 1D). The smooth surface was imperative for achieving reproducible tribological measurements.

### Titanium Anodization

An electrochemical cell containing two-electrodes, i.e., platinum (cathode) and titanium (anode) was used. The electrolyte solution contained 1 M (NH₄)₂SO₄ and 0.5 wt % NH₄F. All electrolytes were prepared from reagent grade chemicals and deionized water. The electrochemical treatment was conducted with a DC power source operated at 2.5 V and 1.5 A, at room temperature for 2.5 h. After the electrochemical treatment, the samples were rinsed with deionized water and dried under nitrogen stream.

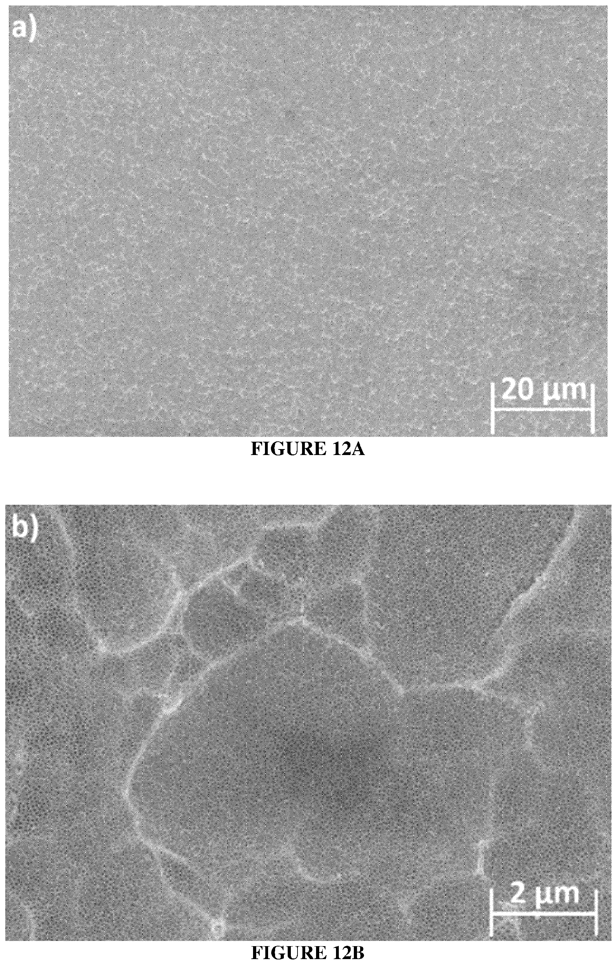

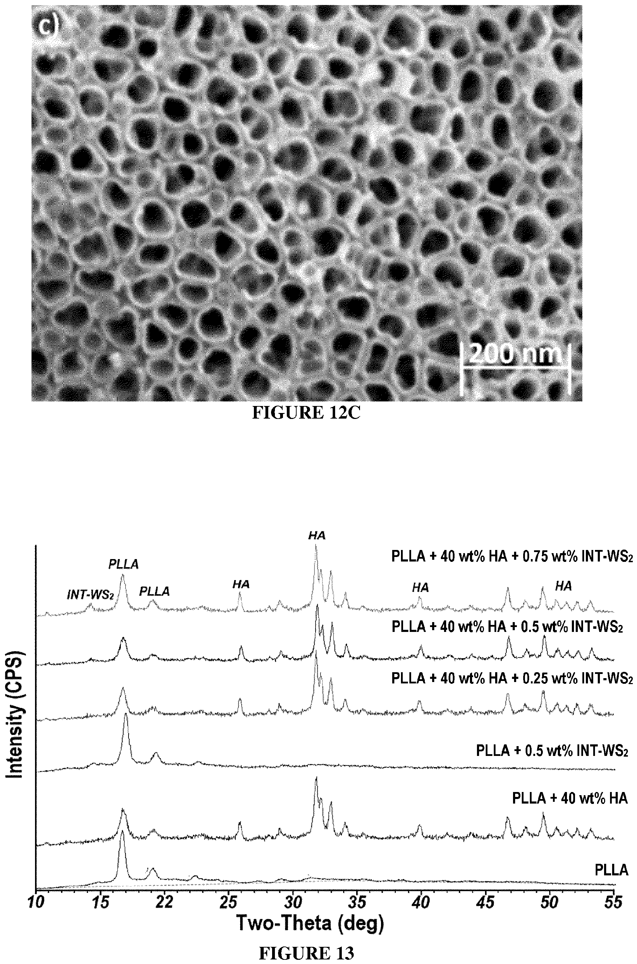

The surface of the titanium after anodization is displayed in Figures 12A-12C. Visibly the anodized titanium surface consists of a dense array of (TiOz) nanotubes with the range of pore diameters between 50-130 nm, which form a highly organized, roughly hexagonal, pattern on the Ti surface.

### Electrophoretic Deposition (EPD)

The detailed synthesis of the Re:IF-MoSz nanoparticles (Re content < 0.1 wt%), which were added to the coating processes, was reported before [Yadgarov, L.; et al. Investigation of Rhenium-Doped MoSz Nanoparticles with Fullerene-Like Structure. Z. Anorg. Allg. Chem. 2012, 638, 2610-2616]. Three different chemical baths were used for electrophoretic deposition of HA + IF NP on the porous titanium substrate. Titanium samples were used as the working electrode (cathode), while a platinum plate served as the anode. The final volume of all three electrolyte solutions containing 1 mg of the IF NP was 50 mL.

***Solution A:*** The electrolyte solution consisted of 42 mM Ca(NO₃)₂ and 25 mM NH₄H₂PO₄, 1 mg Re:IF-MoS₂ sonicated in 3 mM cetyl trimethylammonium bromide (CTAB). Ethyl alcohol was added into the above solution in a 1:1 ratio in order to reduce the hydrogen evolution on the titanium electrode. The initial pH of the electrolyte solution was 4.5. The coating process was carried out at 40 °C with a DC power supply at 20 V bias and 0.11 A for 3 h. The samples were washed with deionized water and dried for 24 h at 100 °C.

***Solution B:*** The electrolyte solution consisted of 5.25 mM Ca(NOs)z, 10.5 mM NH₄H₂PO₄, and 150 mM NaCl. The initial pH of the solution was adjusted to 5.30 by adding NaOH. 1 mg Re:IF-MoS₂ was sonicated in distilled water for 15 min and added to the electrolyte solution. The coating process was conducted with a DC power source operated at 2.5 V and 0.11 A at room temperature for 3 h.

***Solution C:*** The electrolyte solution consisted of 3 mM Ca(NO₃)₂ and 1.8 mM KH₂PO₄, 1 mg Re:IF-MoS₂ sonicated in 3 mM CTAB. The initial pH of the electrolyte solution was 5. The coating process was conducted with a DC power source operated at 6 V and 1 A at room temperature for 1 h. The resulting samples, after coating, were washed with deionized water and dried in room temperature.

The formal molar Ca/P ratio in HA is 5:3 (1.67). The Ca/P ratio in each coating was calculated based on semi-quantitative. Energy dispersive spectroscopy (EDS) analysis. For ***solution A*,** the ratio was found to be 2.6. The higher abundance of calcium in this coating could be attributed to the presence of portlandite (Ca(OH)₂). The Ca/P ratio of the coating obtained from ***solution B,*** which was highly crystalline and discontinuous was 1.5, which agrees well with the HA formula (1.66). The ratio is 1 for the coating obtained from ***solution C*,** which can be ascribed to the presence of calcium pyrophosphate phase (Ca₂(P₂O₇)) in the coating-see XRD analysis (Example 3).

The bath showing the most uniform coating and good adhesion **(*solution A*)** was then further studied by changing the deposition time to 2, 3 and 4 hours and subsequent annealing at 700°C for 1 h.

### Characterization

### High-Resolution Scanning Electron Microscopy (HRSEM) and High-Resolution Transmission Electron Microcopy (HRTEM):

The surface morphology of the titanium samples was analyzed by (HRSEM) (Zeiss Ultra 55) after each step. For topographical information, the secondary electrons were recorded using the SE2 and In-lens detectors. For atomic number contrast the backscattering electron (BSE) detector was used. In order to avoid the sample charging during the analysis, the imaging was done under relatively low accelerating voltage (2-5 kV) and low current. Energy dispersive spectroscopy (EDS) analysis (EDS Bruker XFlash/60mm) of the samples was undertaken as well. The reported results of the EDS were based on standard-less analysis and hence is semi-quantitative in nature.

TEM was performed with a JEOL 2100 microscope (JEOL Ltd., Tokyo, Japan) operating at 200 kV, equipped with a Thermo Fisher EDS analyzer. High-resolution TEM (HRTEM) images were recorded with a Tecnai F30 UT (FEI) microscope (FEI, Eindhoven, the Netherlands) operating a 300 kV. The TEM grids were prepared by dripping an ethanolic solution of the nanoparticles onto a collodion-coated Cu grids.

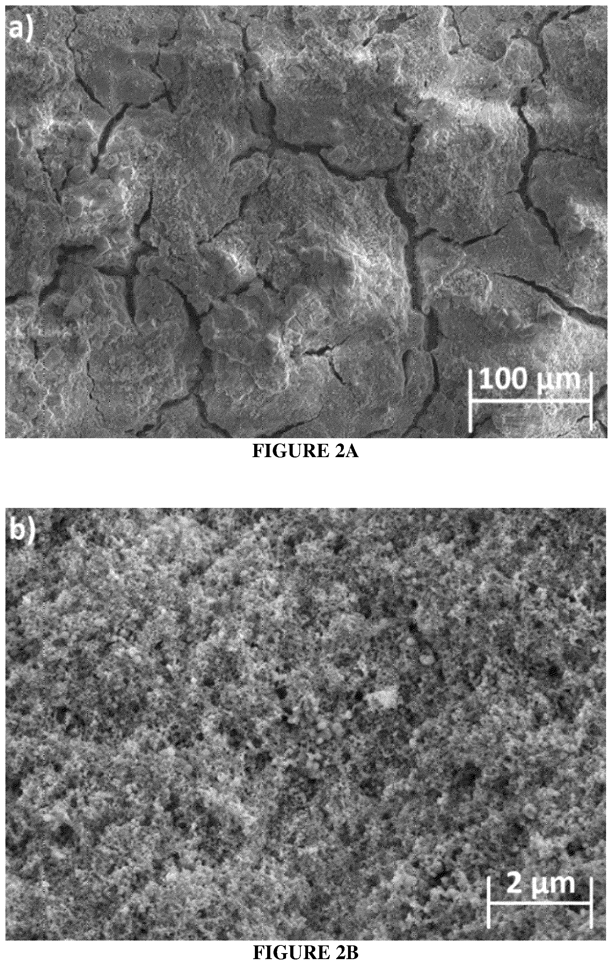

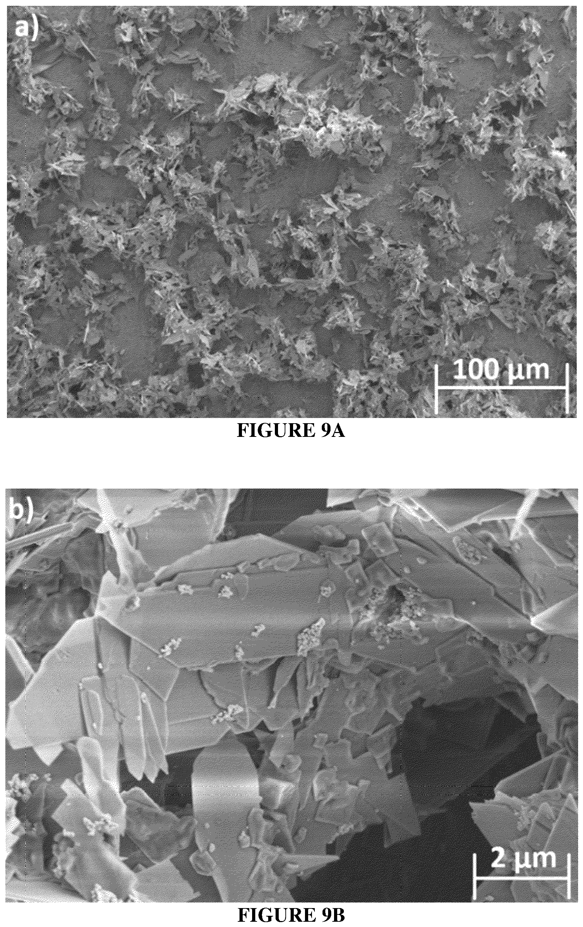

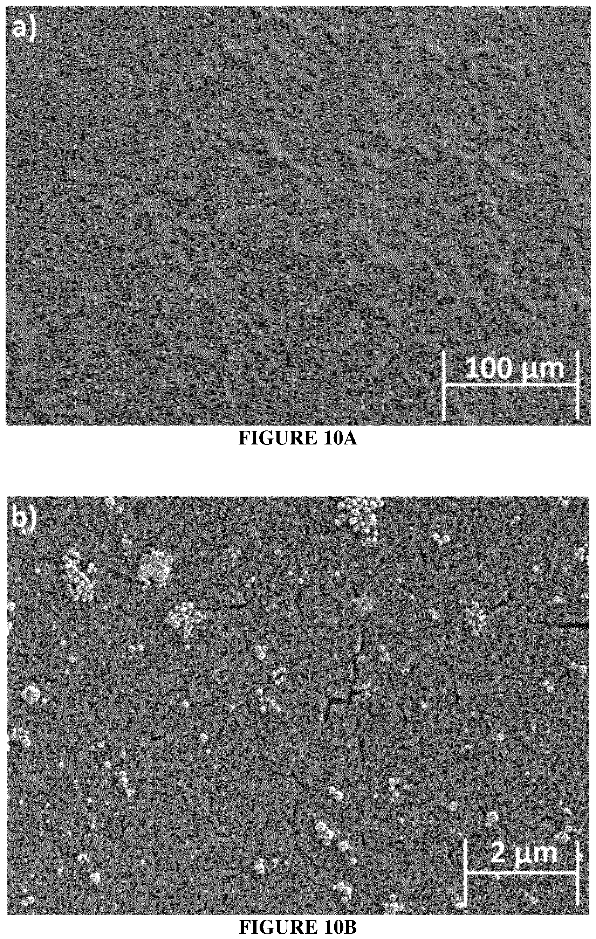

The surface morphology of the HA film prepared via ***solution A*** (Figures 2A-2B) and ***solution C*** was more homogeneous and could be successfully combined with the Re:IF-MoS₂ NP in the films as opposed to the film obtained from ***solution B,*** which was highly crystalline but nonuniform. The surface morphology of the film obtained from ***solutions B*** and **C** are shown in Figures 9A-9B and 10A-10B, respectively

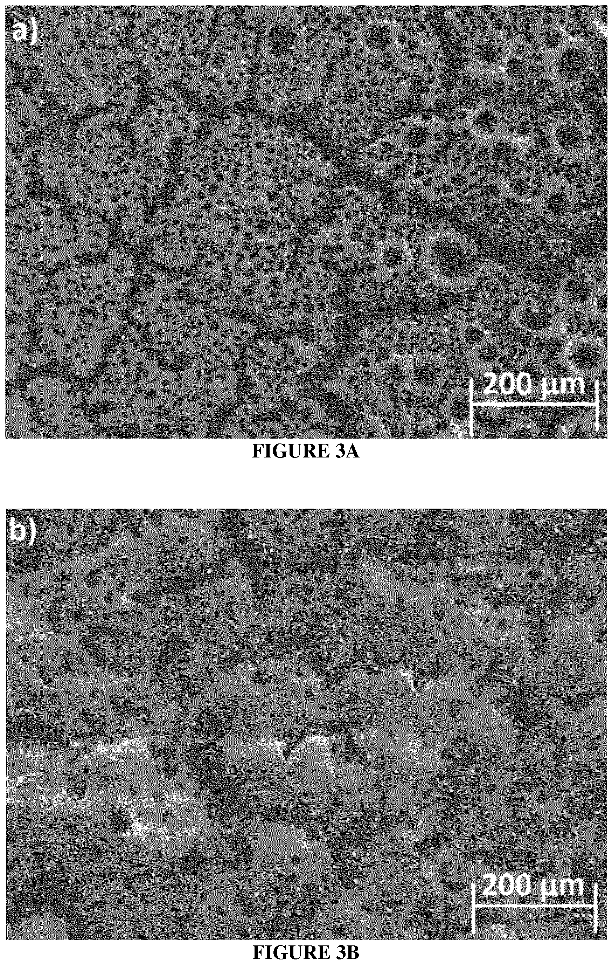

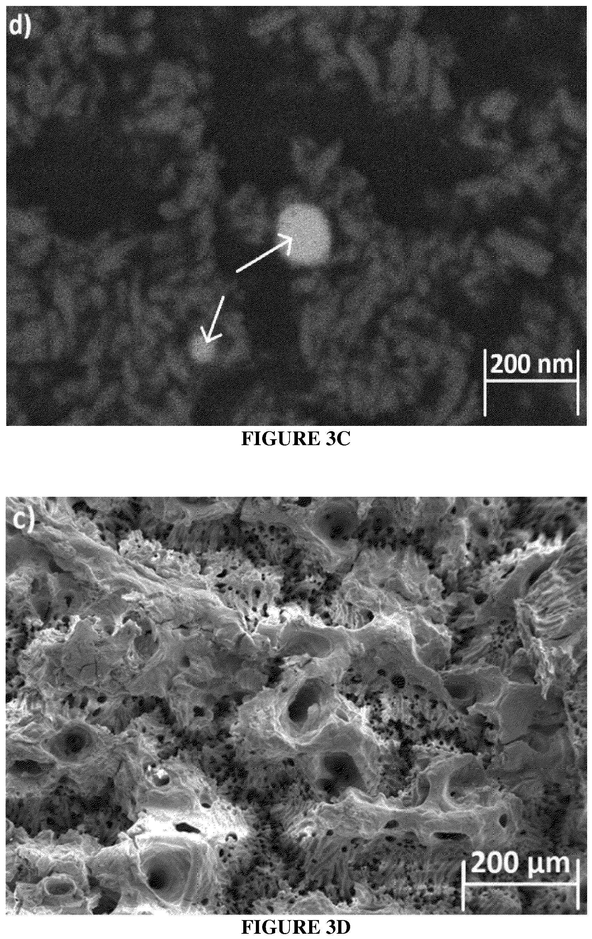

The SEM images of the surface of the HA films with Re:IF-MoS₂ nanoparticles obtained from ***solution A*** for different deposition periods are shown in Figures 3A-3D. The surface of the coated film shows defects, including the presence of cracks and pores with circular shape. Such pores can probably be attributed to the formation of Hz(g) bubbles during the coating process.

Interestingly, the bias applied during EPD for ***solution B*** (and ***C***) was appreciably smaller (2.5 V) compared to ***solution A*** (20 V). On the other hand, the film obtained by EPD from ***solution A*** was quasi-continuous. It was highly crystalline but less uniform in the case of ***solution B,*** i.e., the apparent current density was higher than that calculated on the basis of the formal electrode surface. The higher voltage used for the EPD from ***solution A*** implied a much higher rate of hydrogen production, which could explain the porous structure of this film. The density of the pores and their sizes could be possibly tuned by the bias applied on the cathode during the electrophoretic deposition. Furthermore, addition of surface active agents, like CTAB and others, could reduce the size of the pores.

The large cracks are diminished, and the pore-size decreased as the coating time was prolonged. The thickness of the coating was a few microns, therefore the nanoparticles could have been buried under the film surface and even be closer to the titanium substrate. Using low energy beam (2 keV) in the BSE mode, the IF NP could be nevertheless observed (Figure 3D).

### EXAMPLE 2 (not according to the claimed invention)

### Zeta Potential Results of Hydroxyapatite (HA) and rhenium doped fullerene like MoS₂ (Re:IF-MoS₂) Film on Titanium Substrate

The surface charge of the HA suspension with and without the nanoparticles was determined by zeta potential (ZP) measurements using ZetaSizer Nano ZS (Malvern Instruments Inc., Malvern, UK) with a He-Ne light source (632 nm). To prepare the samples for these measurements, IF (0.6 mg) NP were deagglomerated in 20 mL purified water by sonicating for 5-10 minutes using an ultrasonic bath (see Figure 1C for a SEM image of such an agglomerate). Subsequently, 0.2 mL of the IF suspension was added to 1.5 mL aqueous solutions with pH varying from 1 to 12 and sonicated for an extra 5 min. Before the addition of the IF NP, the pH of each solution was adjusted using concentrated NaOH or HC1. The final concentration of the IF NP was 0.004 mg/mL. The ZP of the solutions was measured in a folded capillary cell (DTS1060) made from polycarbonate with gold plated beryllium/copper electrodes.

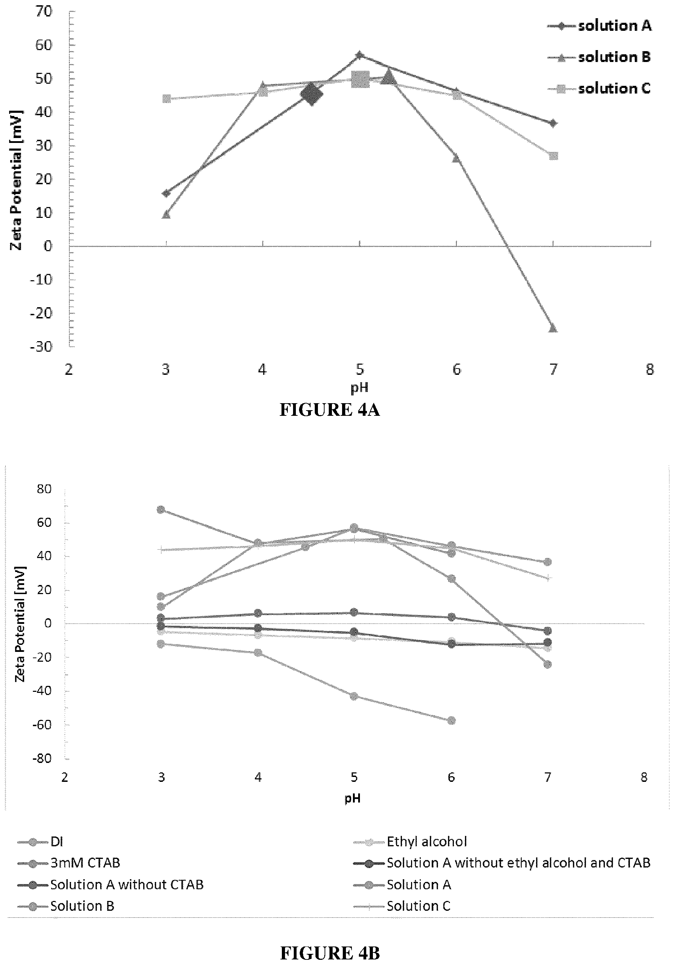

Figures 4A-4B show the results of the Zeta potential (ZP) measurements performed with the three solutions containing Re:IF-MoSz nanoparticles as a function of pH-up to pH=7. The ZP of all the solutions containing the nanoparticles was positive for pH below 6.5. At higher pH the ZP of ***solution B*** became negative, while that of ***solutions A*** and ***C*** remained positive. This difference can be attributed to the addition of the CTAB, which is a cationic surfactant, to ***solutions A*** and ***C*.** The (positive) ZP of the natural solutions used for EPD is marked on Figure 4A for all three solutions.

The ZP measurements showed that the species in the HA solution containing the IF NP were positively charged and consequently, the HA film could be deposited on the negative electrode (Ti) during the EPD process. The ZP of the IF NP in pure water, ethanol solution, CTAB in water, and the three solutions used for the EPD (included also in Figure 4A) are summarized in Figure 4B, the errors of the ZP measurements were about 2%.

### EXAMPLE 3 (not according to the claimed invention)

### X-Ray Diffraction (XRD) of Hydroxyapatite (HA) and rhenium doped fullerene like MoS₂ (Re:IF-MoS₂) Film

The film was removed from the Ti substrate and carefully crushed into a powder. The powder was analyzed by X-ray powder diffraction (XRD) using TTRAX III (Rigaku, Tokyo, Japan) theta-theta diffractometer equipped with a rotating copper anode X-ray tube operating at 50 kV/200 mA. A scintillation detector aligned at the diffracted beam was used after a bent Graphite monochromator. The samples were scanned in specular diffraction mode (θ/2θ scans) from 10 to 80 degrees (2θ) with step size of 0.025 degrees and scan rate of 0.5 degree per minute. Phase identification and quantitative analysis were performed using the Jade 2010 software (MDI) and PDF-4+ (2016) database.

The results of the XRD analyses are summarized in Figures 5A-5B and in Table 1. The XRD patterns of the different coatings obtained from ***solutions A, B and C*** are shown in Figure 5A. The major phase obtained by EPD of these solutions is HA. Nonetheless, the coating obtained from ***solution A*** contained appreciable amounts (25 wt %) of portlandite (Ca(OH)₂). ***Solution B**,* on the other hand, contained, in addition to the HA, also significant amounts of brushite-(CaH(PO₄)·2H₂O). The film obtained from ***solution C*** contained calcium pyrophosphate-(Ca₂(P₂O₇)). The presence of the Re:IF-MoS₂ nanoparticles in the coatings is confirmed by the tiny peak at 14.3°. The content of the IF NP is calculated as 0.2 wt % for ***solution A,*** 1.5 wt % for ***solution B*** and 1.4 wt % for ***solution C***. This amount is rather small but could nevertheless lead to major improvements of the tribological properties of the film without compromising its mechanical robustness.

Following the annealing of the film obtained from ***solution A*** (Figure 5B), the HA became biphasic calcium phosphate (BCP), i.e., intimate mixture of two phases: HA (73.6 wt %) and β-TCP (5.9 wt %), and 0.1 wt % Re:IF-MoS₂ NP.

**Table 1. Composition of the films deposited from different solutions determined from the XRD analysis.**

| **EPD films** | **HA** | **Portlandi te** | **Brushit e** | **Calcium Pyrophosp hate** | **β-TCP** | **Re:IF-MoS₂** |
|---|---|---|---|---|---|---|
| Film obtained from solution A | 74.8 wt % | 25 wt % | | | | 0.2 wt % |
| Film obtained from solution B | 17.2 wt % | | 81.3 wt % | | | 1.5 wt % |
| Film obtained from solution C | 81.1 wt % | | | 17.5 wt % | | 1.4 wt % |
| Film obtained from solution A after annealing | 73.6 wt % | | | 20.4 wt % | 5.9 wt % | 0.1 wt % |

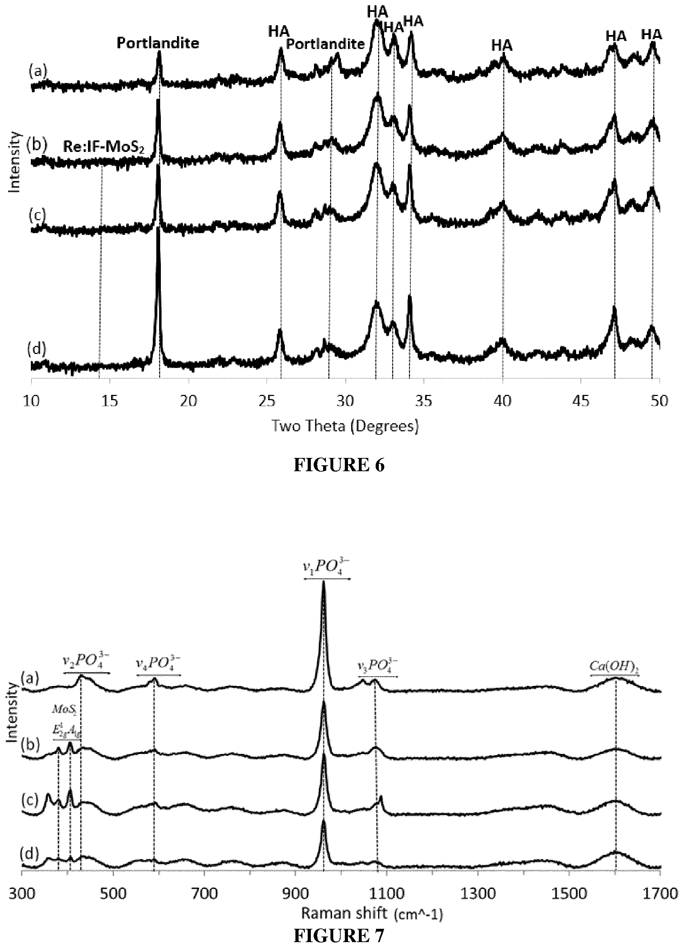

The XRD patterns of the films obtained from ***solution A*** without the NP (a) and with the IF NP for different deposition times (b-c) is shown in Figure 6. The percentages of the compounds in each film is presented in Table 2. The major phase in the films was hydroxyapatite. The relative amount of the portlandite in the film increased with extending deposition times (Figure 6). The relative amount of the calcium oxide did not vary with the deposition time which was also true for the relative content of the IF NP. Although the signal of the IF NP was non-visible in Figure 6, their presence is confirmed through both electron microscopy (Figures 3A-3D) and the Raman measurements (Figure 7).

**Table 2. Composition of the film determined via XRD analysis for different deposition times (from solution A.**

| **EPD films** | **HA** | **Portlandite** | **Calcium Oxide** | **Re:IF-MoS₂** |
|---|---|---|---|---|
| Film obtained from solution A without Re:IF-MoS₂ (3 h) | 87.8 wt % | 4.6 wt % | 7.6 wt % | |
| Film obtained from solution A (2 h) | 82.6 wt % | 7.4 wt % | 9.1 wt % | 0.3 wt % |
| Film obtained from solution A (3 h) | 80.4 wt % | 11.3 wt % | 8.0 wt % | 0.3 wt % |
| Film obtained from solution A (4 h) | 77.8 wt % | 13.6 wt % | 8.3 wt % | 0.3 wt % |

### EXAMPLE 4 (not according to the claimed invention)

### Raman Spectroscopy of Hydroxyapatite (HA) and rhenium doped fullerene like MoS₂ (Re:IF-MoS₂) Film

Raman spectra of the powders ground from the films were obtained with Horiba-Jobin Yivon (Lille, France) LabRAM HR Evolution set-up using solid state laser with a wavelength of 532 nm. The instrument was equipped with Olympus objectives MPlan N 100 × NA 0.9. The measurements were conducted using a 600 grooves/mm grating. Each spectrum was acquired for 20 s and the spectra were averaged 100 times, which enabled using low excitation power thereby preserving the sample integrity. The spectral ranges collected were from 100 to 1800 cm⁻¹.

The Raman spectra of HA + IF films prepared from ***solution A*** for different deposition times (2, 3 and 4 hours) are shown in Figure 7. The spectra showed the characteristic vibration bands of calcium hydroxide (wide peak at 1600 cm⁻¹) and poorly crystalline phosphoric moieties, especially phosphate PO₄⁻³ bands at 469 (vz), 562-603 (ν₄), 962 (vi) and 1000-1104 cm⁻¹ (vs). These bands are typical of HA. The Raman spectra showed also the typical MoS₂ modes at 383 (E_{2g}) and 408 cm⁻¹ (A_{1g}). Interestingly, in contrast to the XRD pattern (Figure 6), the Raman bands of the IF NP in the HA film are easily discerned here.

### EXAMPLE 5 (not according to the claimed invention)

### Tribological Results of Hydroxyapatite (HA) and rhenium doped fullerene like MoS₂ (Re:IF-MoS₂) Film

A home-made ball-on-flat rig was used for the tribological tests. The tests were carried-out at room temperature and humidity of ~40%. Each test was repeated 5-times. Tribological tests were performed on the titanium samples at every step of the experimental procedure. The tribological testing was done under dry friction conditions. This testing method utilizes flat lower samples and a ball-shaped upper specimen, which slides against the flat specimen. The two surfaces move relative to each other in a linear, back and forth sliding motion, under a prescribed set of conditions. In this testing method, the load is applied vertically downwards through the ball against the horizontally mounted flat specimen. Two measurements procedures were used in these series of tests. Sliding speed of 0.3 mm/s was common to both series. In one series of measurements the load was 10 g; the diameter of the ball (hard steel-AISI 301) was 10 mm and consequently a Hertzian pressure of 150 MPa was applied on the film (20 cycles). In another series, the load was 20 g, the diameter of the ball 2 mm, i.e., a Hertzian pressure of 600 MPa was applied, and the number of cycles was 100.

Table 3 summarizes the data for the friction coefficient and surface roughness of the different samples under dry conditions. In general, the friction coefficient was found to go down along with the stages of the experimental procedure of preparing the film. The low friction coefficient of the HA film obtained from ***solution A*** can be attributed to the IF nanoparticle structure. The nanoparticles exhibited facile rolling when released from the film surface. In addition, gradual peeling/crushing of the NP and material transfer from the film surface to the counter surface of the ball contributed to the facile shearing of the mating surfaces and low friction coefficients. Interestingly, the friction coefficient of the HA film obtained from ***solution A*** was maintained also after 700 °C annealing.

**Table 3. Summary of the initial and final friction coefficients and the initial roughness for different stages of preparation of the composite HA + IF film. Measurement conditions: diameter of the test ball 10 mm; load = 10 g (Hertzian pressure-P = 150 MPa).**

| **Tested film** | **Initial Coefficient of Friction** | **Final Coefficient of Friction (after 20 Cycles)** | **Initial Roughness (µm)** |
|---|---|---|---|
| Titanium after surface treatment | 0.50 ± 0.01 | 0.60 ± 0.02 | 0.23 ± 0.03 |
| Titanium after anodization | 0.15 ± 0.01 | 0.23 ± 0.03 | 0.50 ± 0.05 |
| Film of HA with Re:IF-MoS₂ NP obtained from solution A on anodized titanium | 0.11 ± 0.01 | 0.13 ± 0.01 | 0.45 ± 0.4 |
| Film of HA with Re:IF-MoS₂ NP obtained from solution B on anodized titanium | 0.21 ± 0.02 | 0.43 ± 0.08 | 0.37 ± 0.03 |
| Film of HA with Re:IF-MoS₂ NP obtained from solution C on anodized titanium | 0.37 ± 0.23 | 0.30 ± 0.18 | 0.52 ± 0.02 |
| Film of HA with Re:IF-MoS₂ NP obtained from solution A on anodized titanium after annealing | 0.12 ± 0.01 | 0.11 ± 0.02 | 0.49 |
| | | | 0.7 |

Table 4 shows the dry friction coefficient of the coatings obtained from ***solutions A*** without (3 h) and with the NP after 2, 3 and 4 h of deposition time on the anodized titanium substrate. A higher Hertzian pressure (600 MPa) was used for the tribological test. The dry friction coefficient was reduced with increasing coating-time of the film.

Following the 4 h deposition time the friction coefficient was very low (0.12) attesting to the quality of the composite film.

**Table 4. The initial and final friction coefficients and the initial roughness of the coating on titanium substrate obtained from solution A for different periods of deposition. Measurement conditions: diameter of the test ball 2 mm; load 20 g and Hertzian pressure of P = 600 MPa.**

| **Tested film** | **Initial Coefficient of Friction** | **Final Coefficient of Friction (after 100 Cycles)** | **Initial Roughness (µm)** |
|---|---|---|---|
| Pure HA film obtained from solution A without NP after 3 h deposition | 0.66 ± 0.08 | 0.78 ± 0.04 | 1.59 ± 0.28 |
| HA film with Re:IF-MoS₂ NP obtained from solution A after 2 h | 0.75 ± 0.05 | 0.63 ± 0.03 | 0.49 ± 0.05 |
| HA film with Re:IF-MoS₂ NP obtained from solution A after 3 h | 0.53 ± 0.03 | 0.55 ± 0.04 | 0.57 ± 0.17 |
| HA film with Re:IF-MoS₂ NP obtained from solution A after 4 h | 0.13 ± 0.01 | 0.12 ± 0.02 | 0.48 ± 0.02 |

Therefore, it is clear that the extended deposition of the composite film resulted in lower friction under very high load. However, the mechanical stability of the film might have been partially compromised. The surface roughness of the films was in the sub-micron range for all the films containing the NP.

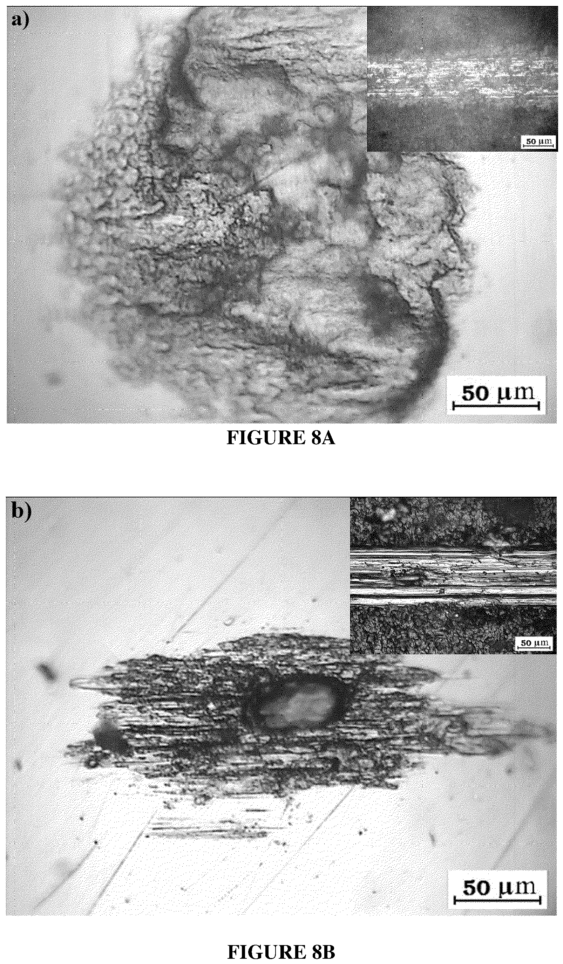

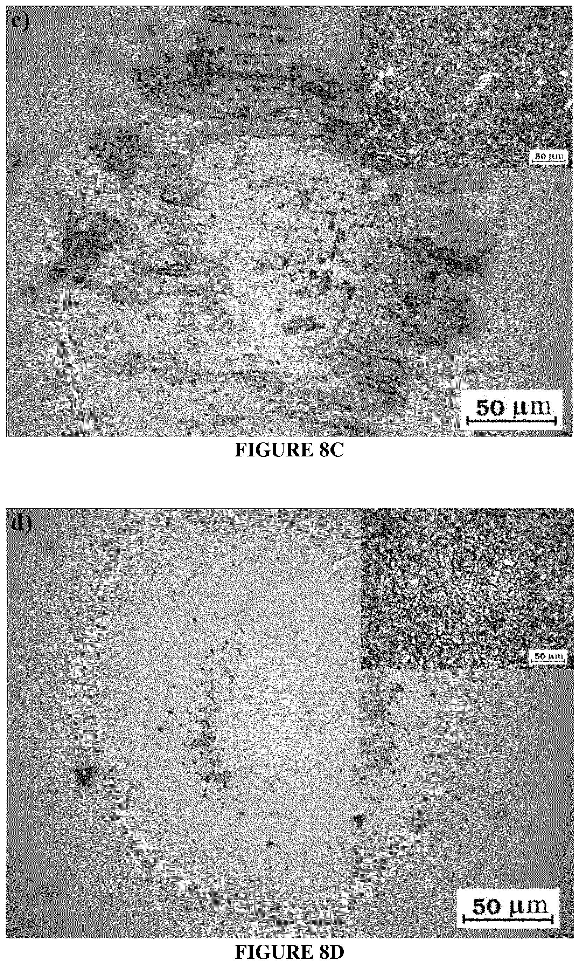

Figures 8A-8D show optical micrographs of the wear of the ball and the wear track on the film (inset) after different periods of EPD (600 MPa) and 100 cycles. In analogy to the friction coefficient, the visible wear scar on the ball and the wear track on the film were markedly reduced with the deposition time of the HA + IF NP film.

### EXAMPLE 6 (not according to the claimed invention)

### Methods for Film Formation

### Sol-gel deposition:

A solution of 3M (C₂H₅O)₃PO was hydrolyzed for 24 h in a sealed container under vigorous stirring, 3 M Ca(NO₃)₂4H₂O was added dropwise with 1 mgr Re:IF-MoS₂ nanoparticles in anhydrous ethanol. The mixed sol solution agitated for additional 30 min and kept static at ambient duration time for 24 h. Ti₆Al₄V substrate was dip coated in the sol solution, then dried at 80 °C and followed by annealing in vacuum at 900 °C for 5 h.

### Dip coating:

3 mM Ca(NO₃)₂, 1.8mM KH₂PO₄ were dissolved in distilled water, then adding 1 mgr Re:IF-MoSz nanoparticles after dispersion. Immersing titanium substrate in the solution at 37 °C and sealied the container for 24 h, finally the substrate was drying at room temperature, followed by annealing at 700 °C for 3 h.

### Casting molding

PLLA was dissolved in dichloromethane and adding hydroxyapatite powder with Re:IF-MoSz nanoparticles to the polymer solution, split the solution to Teflon mold and drying at room temperature.

### EXAMPLE 7

### Film Formation including PLLA, HA and INT-WS₂

### Materials

Poly L-lactic acid (PLLA) with an inherent viscosity midpoint of 2.4 dl/g was purchased from Corbion (Gorinchem, The Netherlands). Oleic acid (OA, ≥99%) and Hydroxyapatite (HA, nanopowder, <200 nm particle size (BET), ≥97%, synthetic) were purchased from Sigma Aldrich Chemical Company.

INT-WS₂ with diameter between 30-150 nm and length between 1-20 micrometer were synthesized using a published procedure [Chithaiah, P.; Ghosh, S.; Idelevich, A.; Rovinsky, L.; Livneh, T.; Zak, A. Solving the "MoSz nanotubes" synthetic enigma and elucidating the route for their catalyst-free and scalable production. ACS Nano 2020, 14, 3004-3016]. Briefly, the precursor nanoparticles of tungsten trioxide grew into high aspect ratio tungsten suboxide nanowhiskers under mild reducing atmosphere at 840 °C. Subsequently, sulfurization of the nanowhiskers result in hollow WS₂ nanotubes.

The PLLA/HA/INT films were prepared by the solvent casting method according to the following procedure.

***PLLA neat films:*** 0.75 g of PLLA pellets was dissolved in 20 ml chloroform and mechanically mixed. Subsequently, the solution was poured onto a Teflon plate for drying in the hood with an aluminum foil cover punctuated with 10 holes.

***PLLA films with 40 wt% hydroxyapatite:*** 0.75 g of PLLA pellets was dissolved in 15 ml chloroform; 300 mg of hydroxyapatite nanoparticles were mixed with 5 ml chloroform and 150 µ1 oleic acid for 30 min. The two solutions were mixed together using a magnetic stirrer for 5 min before pouring onto a Teflon plate and were then dried in the hood using aluminum foil cover punctuated with 10 holes.

***PLLA films with 0.5 wt% INT-WS₂:*** First, 3.8 mg INT-WS₂ powder was dispersed in 5 ml ethanol for 3 min and vacuum annealed for 1.5 h at 80 °C. Next, 0.75 g of PLLA pellets were dissolved in 15 ml chloroform and mechanically mixed for 5 hours; then the annealed INT-WS₂ were dispersed in 5 ml chloroform for 3 min. Finally, the two solutions were mixed together using a magnetic stirrer for 5 min before pouring onto a Teflon plate for drying in the hood with an aluminum foil cover punctuated with 10 holes.

***PLLA films with 40 wt% hydroxyapatite and 0.25, 0.5 and 0.75 wt% LNT-WS₂:*** First, 1.9, 3.8 or 5.6 mg of INT-WS₂ powder were dispersed in 5 ml ethanol for 3 min and vacuum annealed for 1.5 h at 80 °C. Next, 0.75 g PLLA pellets were dissolved in 10 ml chloroform, while 300 mg of hydroxyapatite nanoparticles were dispersed in 5 ml chloroform and 150 µl oleic acid for 30 min. Afterwards, the annealed INT-WS₂ were dispersed in 5 ml chloroform for 3 min. Finally, the three solutions were mixed together using a magnetic stirrer for 5 min before pouring onto a Teflon plate for drying in the hood with aluminum foil cover punctuated with 10 holes.

The dried samples were vacuum annealed for 5 days at 45 °C. The thickness of the films was determined by caliper and was on the average 80 µm for pure PLLA; 113 µm for PLLA+HA; and 93 µm for PLLA+HA+INT. The densification of the films upon the addition of the nanotubes was attributed to alignment of the polymer molecules along the nanotubes surface. The texturing of the polymer molecules was an indirect evidence to the non-specific but nevertheless strong nanotube-polymer interfacial interaction and explained the mechanical reinforcement of the PLLA by the INT.

### EXAMPLE 8

### Characterization of films of this invention comprising PLLA, HA and INT-WS₂

### X-ray Diffraction

Comparison between the XRD patterns of the nano composite films and HA (hydroxyapatite) powder is shown in Figure 13. The XRD pattern of the composite PLLA and HA and INT-WS₂ film contains peaks of the different components, which indicates that the composition and structure of PLLA and HA and nanotubes were not affected by the fabrication process of the film.

Using the Pawley-based WPF analysis, the degree of crystallinity of the samples was calculated by comparing the total area under all the crystal peaks and the area under the amorphous halo. The results are presented in Table 1. The degree of crystallinity of the PLLA film was calculated to be 32.8%. After adding 0.5 wt% of INT-WS₂ the degree of crystallinity is slightly increased to 33.2%. However, after adding HA nanoparticles to the PLLA film, the degree of crystallinity increased significantly to 37.1% and remained essentially the same even after adding 0.25-0.75 wt% of INT-WS₂. The average crystallite size of the different compositions was estimated using the Williamson-Hall approach from the XRD peak widths fitted in the Pawley-based WPF analysis and the Scherrer equation using the main peak of the PLLA at 16.5° and is reported also in Table 5. The largest crystallite size (171 Å) occurred for the neat PLLA film. As expected, the foreign ingredients (HA and INT) served as crystallization nuclei for the PLLA and reduced its average crystallite size.

**Table 5. The degree of crystallinity of PLLA film and HA/INT-WS₂/PLLA nanocomposites.**

| **Sample type** | **Degree of crystallinity [%]** | **Average crystallite size [Å]** | | |
|---|---|---|---|---|
| | **WPF** | **Scherrer** | **WPF** | |
| | | | PLLA | HA |
| PLLA film | 32.8 ± 0.9 | 170 | 171 ± 2 | - |
| PLLA film with 40 wt% HA | 37.1 ± 3.0 | 130 | 128 ± 5 | 291 ± 18 |
| PLLA film with 0.5 wt% INT-WS₂ | 33.2 ± 1.6 | 165 | 162 ± 2 | - |
| PLLA film with 40 wt% HA and 0.25 wt% INT-WS₂ | 38.2 ± 2.5 | 145 | 145 ± 5 | 331 ± 20 |
| PLLA film with 40 wt% HA and 0.5 wt% INT-WS₂ | 42.7 ± 2.7 | 140 | 144 ± 4 | 353 ± 19 |
| PLLA film with 40 wt% HA and 0.75 wt% INT-WS₂ | 42.1 ± 2s.2 | 150 | 145 ± 4 | 342 ± 16 |

### High-Resolution Scanning Electron Microscopy (HR-SEM)

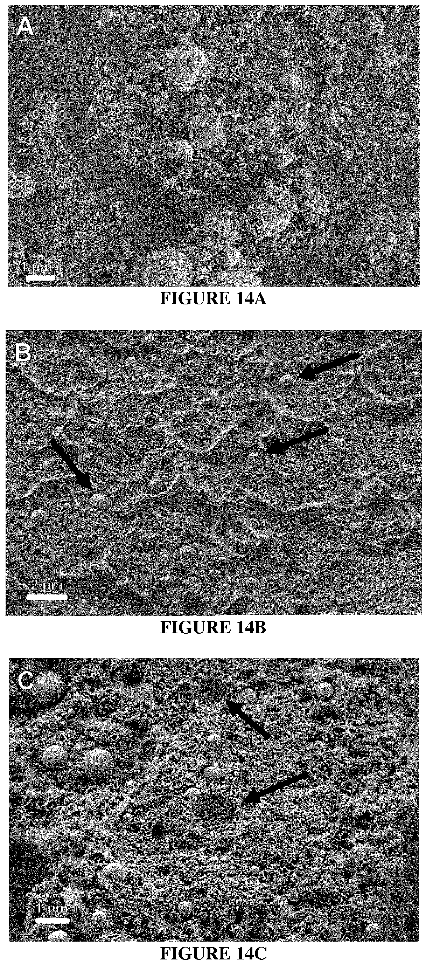

**Figures 14A-14C** show the SEM images of HA powder **(****Figure 14A****),** and a cross-section of PLLA with 40 wt% HA film in secondary electrons (SE) mode **(****Figure 14B** and **14C****).** Visibly **(****Figure 14A****),** the HA particles constitute a bimodal size-distribution made of micron-size agglomerates and a majority phase of a well-dispersed HA nanoparticles (<50 nm). The surface of the large agglomerates is decorated with the small HA NPs. However, the same agglomerates appeared to have a smooth and uniform surface, i.e. free of the decorating HA NPs after being incorporated into the polymer (**Figures 14B** and **14C****-** marked with green arrows). To further understand this effect, the HA phase was washed in ultrasonic bath with the polymer-free solvent (chloroform) containing the oleic acid. It was found that the surface of the large spherical HA agglomerates became smooth and free of the HA NPs decoration after this washing procedure. Therefore, the solvent treatment seem to be responsible for the "cleaning" of the HA spherical agglomerates. These smooth spherical agglomerates of HA are likely to impair the mechanical properties of the film.

**Figures 14B, 14C** show that the HA nanoparticles (NPs) were well dispersed in the polymer matrix, i.e. no phase separation or excessive additional agglomeration was observed, which was not the case in the absence of oleic acid. Furthermore, **Figure 14C** shows that the HA agglomerates (> 0.5 µm) were not damaged during the film breaking process, but were uprooted as a whole from the polymer matrix surface. Furthermore, the hemispherical depressions in **Figure 14C** (red arrows) are indicative of entire HA agglomerates, which were uprooted from the polymer matrix during fracture, possibly being stuck to the other surface of the broken contact. Consequently, one can conclude that the strain was not well transferred to these agglomerates during fracture and hence they adversely affected the mechanical strength of the film.

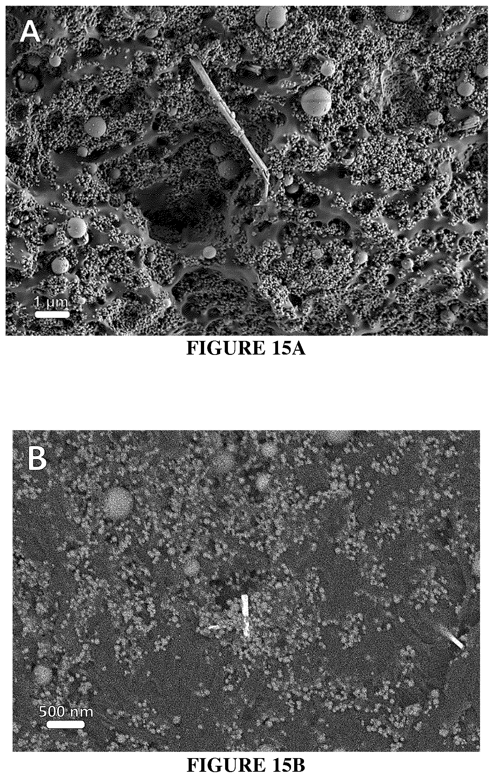

HR-SEM images of the cross section of PLLA reinforced with 40 wt% HA and 0.75 wt% INT-WS₂ are presented in **Figures 15A-15B****.** It can be seen in **Figure 15A** that there was no phase separation and consequently a very good compatibility between the nanotubes and the PLLA matrix and between the HA NPs and nanotubes. The nanotubes protrude from the broken surface, which indicates that they carry some of the applied stress transferred to them from the matrix. In addition, other similar scans show that the INT-WS₂, are fully dispersed in the PLLA matrix.

Visibly, the nanotubes protrude from the PLLA matrix, suggesting that they reinforce the polymer via a bridging and pullout mechanisms .

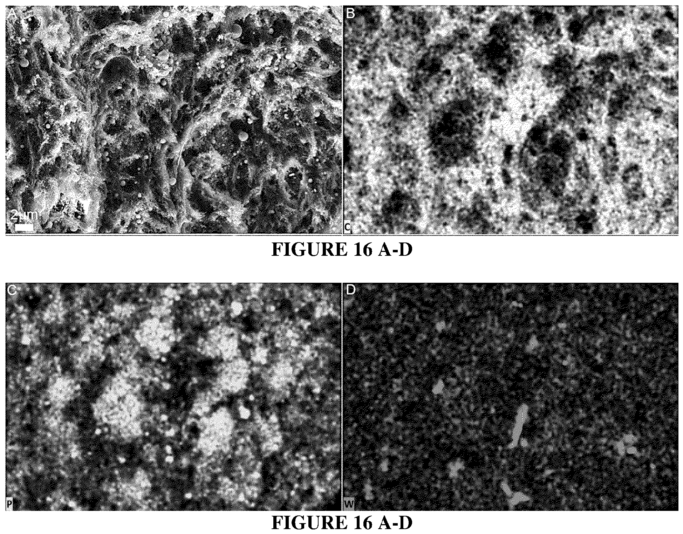

EDS elemental mappings of the PLLA film with 40 wt% HA and 0.75 wt% INT-WS₂ are presented in **Figures 16A-16D****.** The carbon mapping, displayed in **Figure 16B****,** shows that the strong carbon signal was evenly distributed throughout the film. This observation reflects the fact that the matrix of the material was PLLA whose chemical composition was mostly carbon. **Figure 16C** presents the phosphorus mapping, which was a major component of HA. It can be seen that the HA NPs were well dispersed throughout the film. However, the bimodal distribution with distinct micron and submicron-sized spherical agglomerated and evenly distributed HA nanoparticles was clearly discernable. The INT-WS₂ distribution are represented by the tungsten mapping in **Figure 16D****,** which showed that the nanotubes were well dispersed in the PLLA matrix.

### Tensile test

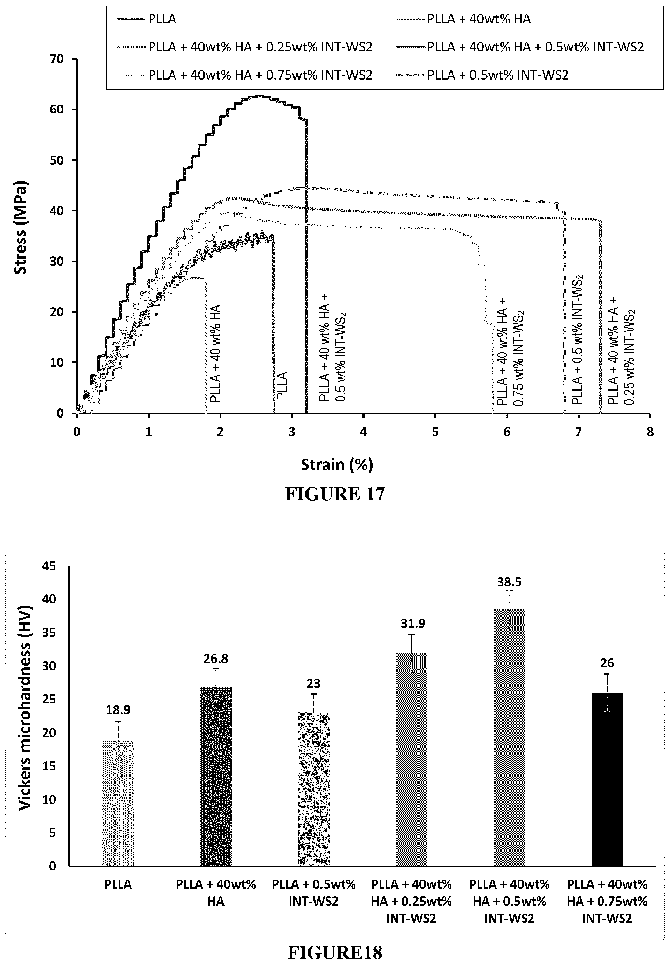

The mechanical properties derived from the stress-strain curves of the films are displayed in **Figure 17** and presented in Table 6.

The Young's modulus of PLLA film with 40 wt% HA (2.4 GPa) increased 1.5 times compared to the neat PLLA film (1.55 GPa), while the yield strength (26.7 MPa) and strain at failure (2.1%) of PLLA film with 40 wt% HA decreased to 0.85 and 0.75 of their values, respectively. Therefore, the toughness of PLLA film with 40 wt% HA (0.3 MPa) was reduced by half compared to that of neat PLLA film (0.6 MPa). This is not surprising, since the HA is an oxide with a small strain to failure. Also, the binding of the HA to the PLLA is not chemical in nature and is rather weak (mostly van der Waals and polar interactions). These two factors adversely affected the fracture toughness of the composite. However, the indentation hardness and modulus of the PLLA+HA composite was appreciably higher than that of pure PLLA (see below). Obviously, the most rational way to mediate between the HA and the PLLA phases and increase the mechanical properties of the nanocomposite would be through surface functionalization. The surface functionalization showed chemical versatility and biocompatibility in order to permit the three constituents (PLLA, HA and INT-WS₂) to optimally interact with each other and exhibit no biotoxicity effects.

The Young's modulus and yield strength of PLLA film with 0.5 wt% INT-WS₂ (2.25 GPa and 44.6 MPa, respectively) increased 1.45 times compared to the neat PLLA film, while the strain at failure of the film with 0.5 wt% INT-WS₂ (6.8%) increased 2.5 times. Therefore, the toughness of the PLLA film with 0.5 wt% INT-WS₂ (2.4 MPa) increased significantly by 4 times compared to the toughness of the neat PLLA film.

The Young's modulus of the PLLA film with 40 wt% HA and 0.5 wt% of INT-WS₂ (3.8 GPa) increased up to 1.7 times compared to the PLLA film with 40 wt% HA and to the PLLA film with 0.5 wt% INT-WS₂. The yield strength of the PLLA film with 40 wt% HA and 0.5 wt% of INT-WS₂ (62.7 MPa) increased by 2.35 and 1.4 times compared to the PLLA film with 40 wt% HA and to the PLLA film with 0.5 wt% INT-WS₂. The strain at failure of the PLLA film with 40 wt% HA and 0.5 wt% of INT-WS₂ (3.2%) increased 1.5 times compared to the PLLA film with 40 wt% HA. However, the PLLA film with 0.5 wt% INT-WS₂, had strain at failure only half the value of the PLLA film with 40 wt% HA and 0.5 wt% of INT-WS₂. Therefore, the toughness of the PLLA film with 40 wt% HA and 0.5 wt% INT-WSz (1.4 MPa) increased significantly by 4.7 times compared to the PLLA film with 40 wt% HA and decreased to 0.6 times the value of the PLLA film with 0.5 wt% INT-WS₂.

**Table 6. The mechanical properties of PLLA film and HA/INT-WS₂/PLLA nanocomposites from tensile testing.**

| **Sample type** | **Young's Modulus (GPa)** | **Yield Strength (MPa)** | **Strain at failure (%)** | **Toughness (MPa)** |
|---|---|---|---|---|
| PLLA film | 1.55 ± 0.15 | 31.0 ± 2.4 | 2.7 ± 1.3 | 0.6 ± 0.2 |
| PLLA film with 40 wt% HA | 2.4 ± 0.1 | 26.7 ± 1.1 | 2.1 ± 0.1 | 0.3 ± 0.1 |
| PLLA film with 0.5 wt% INT-WS₂ | 2.25 ± 0.2 | 44.6 ± 4.65 | 6.8 ± 1.0 | 2.4 ± 0.5 |
| PLLA film with 40 wt% HA and 0.25 wt% INT-WS₂ | 2.7 ± 0.4 | 42.5 ± 5.8 | 7.3 ± 1.0 | 2.6 ± 0.3 |
| PLLA film with 40 wt% HA and 0.5 wt% INT-WS₂ | 3.8 ± 0.5 | 62.7 ± 1.2 | 3.2 ± 1.6 | 1.4 ± 0.7 |
| PLLA film with 40 wt% HA and 0.75 wt% INT-WS₂ | 2.7 ± 0.35 | 39.6 ± 4.9 | 5.8 ± 0.7 | 1.8 ± 0.25 |

### Micro hardness test

**Figure 18** shows the results of the micro-hardness test of PLLA film and the PLLA/HA/INT-WS₂ nanocomposites.

The addition of HA nanoparticles to the PLLA film increased the hardness value (26.8 HV) by 1.4 times compared to the hardness value of the neat PLLA film (18.9 HV). In addition, the hardness of the PLLA film with 0.5 wt% INT-WS₂ (23 HV) increased 1.2 times compared to the hardness of the neat PLLA film.

A more significant increase in the hardness was achieved with the combination of HA and INT-WSz in PLLA. The optimum hardness value was obtained for the films containing PLLA with 40 wt% HA NPs and 0.5 wt% INT-WS₂ with 38.5 HV, a value that is two-times higher than the hardness of the pure PLLA film. It can be deduced that a small amount of nanotubes added to the matrix bridges the gap between the HA nanoparticles creating a uniform network of hardening material. Beyond the optimal concentration of 0.5 wt%, the nanotubes have a deleterious effect on the hardness of the nanocomposite, likely due to agglomeration.

### Nanomechanical Testing (nanoindentation tests)

While microhardness measurements provided an average hardness value, the domain size in the present nanocomposite call for a more local measurements, which will report on possible inhomogeneities in the film. The results from the nanoindentation analysis are presented in Table 7. The results of the nanoindentation experiments are consistent with the Vickers micro-hardness tests discussed above.

The addition of 0.5 wt% INT-WS₂ to the PLLA film caused almost no change in the Young's modulus (3.4 GPa) and hardness (0.18 GPa) values compared to the parameters of the neat PLLA film with Young's modulus of 3.3 GPa and harness of 0.16 GPa. However, the Young's modulus and hardness of the PLLA film with 40 wt% HA (4.9 GPa, 0.24 GPa) increased by 1.5 times each compared to the Young's modulus and hardness of the neat PLLA film. The addition of a small amount of nanotubes to the PLLA film with HA increased the Young's modulus and hardness significantly with the optimum being the addition of 0.25 wt% INT-WS₂. The Young's modulus and hardness of the PLLA film with 40 wt% HA and 0.25 wt% INT-WS₂ (5.6 GPa, 0.36 GPa) increased significantly by 1.7 and 2.25 times, respectively, compared with the values of the neat PLLA film and even the PLLA+HA. Notwithstanding the large fraction of the HA in the film (40 wt%), the hardness values measured here are more than an order of magnitude lower than those reported for a pure HA single crystal. It can be assumed that the difference between the value of hardness measured here and the value observed in the literature originates from the presence of HA NP agglomerates, which degrade the mechanical properties of the material. Obviously also, the mechanical properties of the nanocomposite are compromised due to the weak links between the HA and the PLLA.

Larger statistical variations for some of the composite samples were consistent with local inhomogeneities in the nanoparticle distribution, as is supported by the EDS measurements and mapping, and the Raman studies (below). Nanoindentation results show relative uncertainties an order of magnitude higher in comparison with the microhardness data. This can be attributed to the scale of the inhomogeneities within the sample: EDS mappings in Figure 16 show HA "pockets" of several µm extent, and WS₂ inhomogeneities on a smaller scale. The area of the microindentation imprint varies between 1000 - 2500 µm² (axial length of 30- 50 µm) whereas for the nanoindentations the relevant indentation size is on the scale of the HA pockets, and INT length, 4-5 µm.

**Table 7. Parameters determined from the nanoindentation of PLLA film and HA/INT-WS₂/PLLA nanocomposites.**

| **Sample type** | **Young's Modulus (GPa)** | **Hardness (GPa)** |
|---|---|---|
| PLLA film | 3.3 ± 0.4 | 0.16 ± 0.05 |
| PLLA film with 40 wt% HA | 4.9 ± 0.7 | 0.24 ± 0.06 |
| PLLA film with 0.5 wt% INT-WS₂ | 3.4 ± 0.7 | 0.18 ± 0.08 |
| PLLA film with 40 wt% HA and 0.25 wt% INT-WS₂ | 5.6 ± 1.2 | 0.36 ± 0.15 |
| PLLA film with 40 wt% HA and 0.5 wt% INT-WS₂ | 4.6 ± 0.8 | 0.25 ± 0.08 |
| PLLA film with 40 wt% HA and 0.75 wt% INT-WS₂ | 4.3 ± 0.6 | 0.22 ± 0.07 |

### Thermal properties of PLLA film and HA/INT-WS₂/PLLA nanocomposites by DSC

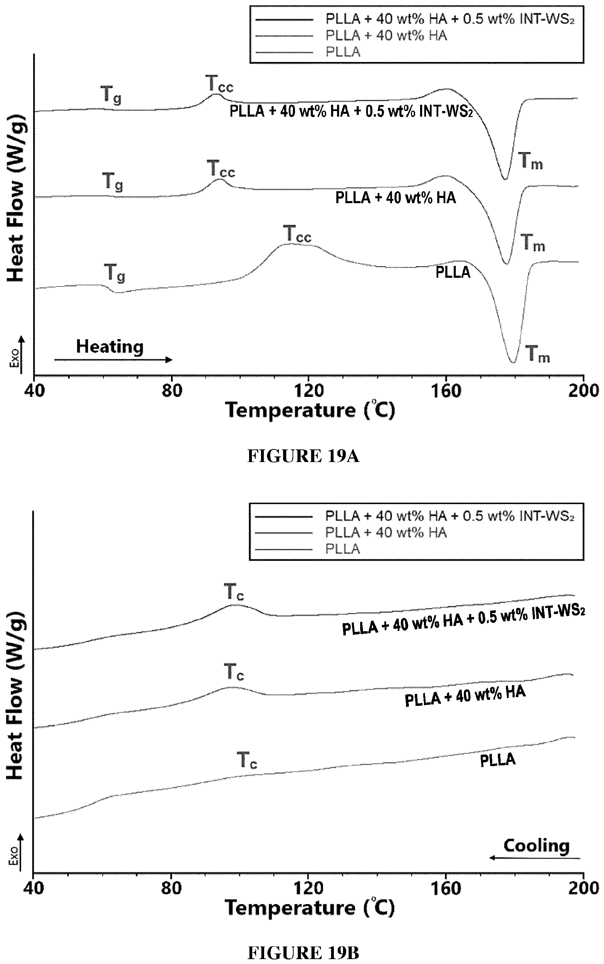

The thermal behavior of the different PLLA films and PLLA/HA/INT-WS₂ nanocomposites films were measured using DSC. The results are summarized in Table 8, and the heating and cooling curves are presented in Figure 19.

The addition of 40 wt% HA nanoparticles to the PLLA film increased the glass transition temperature-T_{g} (62.7 °C) by merely 1.9% compared to the neat PLLA film (61.5 °C). The addition of 0.5 wt% INT-WS₂ to the PLLA film increased T_{g} (66.7 °C) significantly by 8.5% compared to the T_{g} of the neat PLLA film. Therefore, the PLLA film with 0.5 wt% INT-WS₂ has the highest thermal deformation resistance of the films tested.

The cold crystallization temperature - T_{cc} of the PLLA film with 0.5 wt% INT-WS₂ (107.9 °C) is lower than the T_{cc} of the neat PLLA film (114.1 °C). In addition, the PLLA film with 40 wt% HA has lower T_{cc} (93.6 °C) than both the neat PLLA film and the PLLA film with 0.5 wt% INT-WS₂, which indicates that the PLLA with 40 wt% HA NPs film consists of smaller crystallites, compared to the neat PLLA film. The lower ΔH_{cc} of PLLA film with 40 wt% HA and PLLA film with 0.5 wt% INT-WS₂ compared to the neat PLLA film also indicates the presence of bigger PLLA crystallites in the neat PLLA film, which is consistent with the findings from XRD (see discussion of Table 5 data, above).

The addition of 40 wt% HA nanoparticles to the PLLA reduced Tₘ (177.6 °C) compared to the neat PLLA film (179.6 °C), while the addition of 0.5 wt% INT-WS₂ resulted in increased Tₘ (181.7 °C). Therefore, the PLLA film with 0.5 wt% INT-WS₂ has the highest thermal stability. The ΔHₘ values of the PLLA films with 40 wt% HA (33.2 J/g) and PLLA film with 0.5 wt% INT-WS₂ (34.3 J/g) are lower compared to the PLLA film (39.1 J/g). Therefore, the HA nanoparticles and the INT-WS₂ each independently lower the energy required for breaking the polymer chain-chain interactions. The lower T_{c} and higher ΔH_{c} of PLLA film with 40 wt% HA (96.9 °C, 5.6 J/g) compared to the PLLA film (101.6 °C, 2.0 J/g), shows that the PLLA with 40 wt% HA film has a higher cooling rate and smaller crystal nuclei. The higher T_{c} and higher ΔH_{c} of PLLA film with 0.5 wt% INT-WS₂ (116.9 °C, 34.9 J/g) compared to the PLLA film, indicate that the PLLA film with 0.5 wt% INT-WS₂ had lower cooling rate and even smaller crystal nuclei.

The degree of crystallinity - X_{c} and (1-λ)_{c} of the PLLA film with 40 wt% HA (32.2% , 6.0%) was higher compared to the neat PLLA film (7.5% , 2.2%), which indicated that the PLLA film with 40 wt% HA was harder and denser than the neat PLLA film. However, the X_{c} and (1-λ)_{c} of PLLA film with 0.5 wt% INT-WS₂ (33.5% , 36.7%) was even higher compared to the PLLA film with 40 wt% HA, therefore, the PLLA film with 0.5 wt% INT-WS₂ was the hardest and the densest film among the three.

However, the results of micro-hardness test and the nanoindentation tests, show that the hardest film among the three was not the PLLA film with 0.5 wt% INT-WS₂, but the PLLA film with 40 wt% HA. The reason for the difference between the estimated hardness trend and the mechanical measurements could possibly be linked to the nuclei size. The crystallites of the PLLA film with 0.5 wt% INT-WS₂ were larger than the crystallites of the PLLA film with 40 wt% HA. Although not directly relevant, these results are consistent with the Hall-Petch effect, usually associated with polycrystalline metallic films. According to this law, as the size of the crystallites is reduced, the area of their grain boundaries increase, thereby increasing the hardness of the material.

PLLA films with 40 wt% HA and 0.25-0.75 wt% INT-WS₂ have thermal properties (T_{g}, T_{cc}, Tₘ and T_{c}) similar to the PLLA film with 40 wt% HA. Consequently, the PLLA film with 40 wt% HA and 0.25-0.75 wt% INT-WS₂ have smaller thermal deformation resistance, crystallite size, thermal stability and lower cooling rate compared with the PLLA film with 0.5 wt% INT-WS₂. However, the PLLA film with 40 wt% HA and 0.25-0.75 wt% INT-WSz had better thermal deformation resistance, smaller crystallites, smaller thermal stability and lower cooling rate compared to the neat PLLA film.

The ΔH_{cc} of PLLA film with 40 wt% HA and 0.5-0.75 wt% INT-WS₂ (2.5-2.4 J/g) was lower compared to the other samples, which was attributed to the smaller crystallites in the nanocomposite films, due to the combined addition of HA nanoparticles and INT-WS₂ to the PLLA film. The PLLA film with 40 wt% HA and 0.25 wt% INT-WSz had lower ΔH_{cc} (4.8 J/g) compared to the neat PLLA film and higher ΔH_{cc} compared to the rest of the samples. This data demonstrates that addition of a small amount of INT-WS₂ combined with 40 wt% HA produced smaller crystallites compared to the neat PLLA film. The ΔHₘ of the PLLA film with 40 wt% HA and 0.25-0.75 wt% INT-WS₂ was lower compared to the other samples, thus the combined addition of HA nanoparticles and INT-WS₂ to the PLLA film decreased the flexibility of the polymer chains and the energy required to break the interaction between the polymer chains.

PLLA films with 40 wt% HA and 0.25-0.75 wt% INT-WS₂ had similar X_{c} and (1-λ)_{c} to PLLA film with 40 wt% HA, but lower X_{c} and (1-λ)_{c} compared to the PLLA film with 0.5 wt% INT-WS₂. The X_{c} and (1-λ)_{c} of PLLA films with 40 wt% HA and 0.5 wt% and 0.75% INT-WS₂ were very similar, thus they are equally hard. However, the PLLA film with 40 wt% HA and 0.25 wt% INT-WSz had lower X_{c} but higher (1-λ)_{c} compared to the PLLA film with 40 wt% HA and 0.5-0.75 wt% INT-WS₂. Therefore, the PLLA film with 40 wt% HA and 0.25 wt% INT-WS₂ was more elastic but not as hard as the PLLA films with 40 wt% HA and 0.5-0.75 wt% INT-WS₂. This is in agreement with the results of the mechanical properties.

**Table 8. Thermal properties of PLLA film and HA/INT-WS₂/PLLA nanocomposites.**

| **Sample type** | **Tg [°C]** | **Tcc [°C]** | **ΔHcc [J/g]** | **Tm [°C]** | **ΔHm [J/g]** | **Tc [°C]** | **ΔHc [J/g]** | **Xc [%]** | **(1-λ)c [%]** |
|---|---|---|---|---|---|---|---|---|---|
| PLLA film | 61.5 | 114.1 | 32.1 | 179.6 | 39.1 | 101.6 | 2.0 | 7.5 | 2.2 |
| PLLA film with 40 wt% HA | 62.7 | 93.6 | 3.2 | 177.6 | 33.2 | 96.9 | 5.6 | 32.2 | 6.0 |
| PLLA film with 0.5 wt% INT-WS₂ | 66.7 | 107.9 | 3.1 | 181.7 | 34.3 | 116.9 | 34.2 | 33.5 | 36.7 |
| PLLA film with 40 wt% HA and 0.25 wt% INT-WS₂ | 62.9 | 93.5 | 4.8 | 177.3 | 31.1 | 97.2 | 8.5 | 28.3 | 9.2 |
| PLLA film with 40 wt% HA and 0.5 wt% INT-WS₂ | 62.9 | 92.6 | 2.5 | 177.2 | 32.6 | 98.3 | 5.5 | 32.3 | 5.9 |
| PLLA film with 40 wt% HA and 0.75 wt% INT-WS₂ | 62.6 | 95.2 | 2.4 | 177.0 | 32.3 | 99.3 | 5.0 | 32.1 | 5.4 |

### Micro-Raman spectroscopy

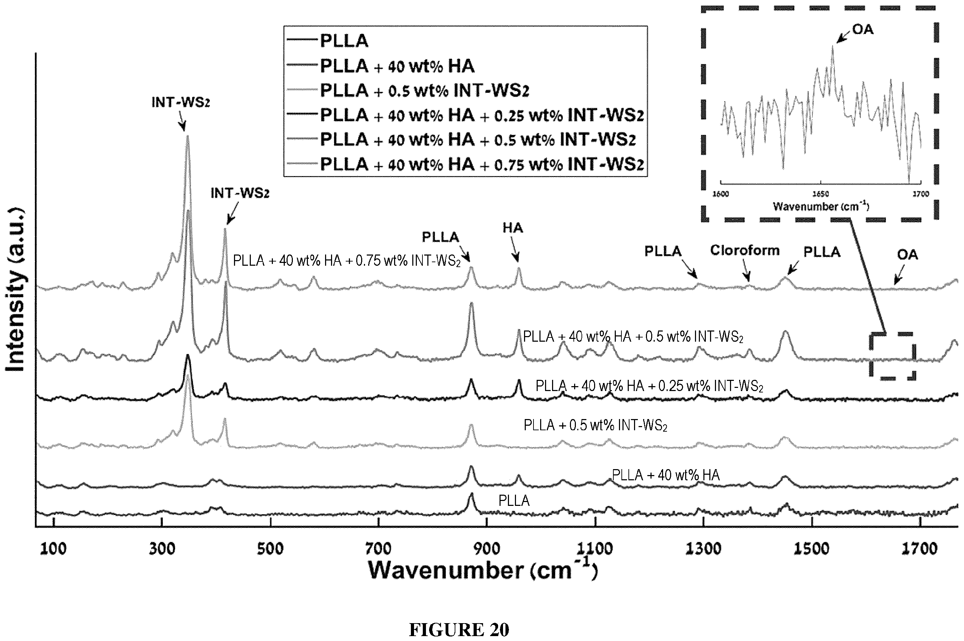

The Raman spectra of the different PLLA films and PLLA/HA/INT-WS₂ nanocomposite films are presented in Figure 20. The PLLA film with 40 wt% HA and the neat PLLA film had exactly the same pattern of peaks and at the same energy (873 cm⁻¹, 1452 cm⁻¹), except the peak of the HA at 960 cm⁻¹. In addition, comparing the PLLA film with 0.5 wt% INT-WS₂ to the neat PLLA film also showed the same pattern of peaks and intensity, except the peaks of the INT-WS₂ at 350 cm⁻¹ and 418 cm⁻¹ . The match between the different spectra patterns was excellent, indicating that no chemical reaction took place between the different ingredients of the nanocomposite, as all the identified peaks belong to the pure reagents, with no missing peaks. Hence the chemical composition of the PLLA was not affected by the addition of the HA NPs and INT-WS₂, or from the production process of the film as suggested above.

The band of 1379 cm⁻¹ was associated with chloroform. That band was seen in the spectra of all the different PLLA films and PLLA/HA/INT-WS₂ nanocomposites films. The existence of this peak indicated that residual amounts of the solvent remained in the films.

The film of PLLA with 0.5 wt% INT-WS₂ and the films of PLLA with 40 wt% HA and 0.25-0.75 wt% INT-WS₂, present peaks at 350 cm⁻¹ and 418 cm⁻¹, which are associated with the E_{2g} and A_{1g} modes of the INT-WS₂.

Oleic acid was a componnent which was incorporated only into the films of PLLA with 40 wt% HA and 0.25-0.75 wt% INT-WS₂. However, the main peak associated with the oleic acid at 1655 cm⁻¹ is rather small and can be observed by focusing on the portion of the spectrum near this peak ( dashed square) and magnifying the scale. The low intensity of the peak reflects the fact that the oleic acid concentration is very low in the films (150 µl).

Raman intensity mapping of the PLLA with 40 wt% HA and 0.5 wt% INT-WS₂ films were carried out (not shown).

Intensity mapping of the PLLA peak at 873 cm⁻¹ showed a relatively uniform Raman light scattering intensity on the entire scanned area, with minimum value of 60% with respect to the maximum (normalized) intensity. This indicates, as suggested above, that the PLLA film was uniform and that it was not affected by the addition of the solvent, HA, and/or INT-WS₂, nor from the fabrication process of the film. The result showed also that no chemical reactions occurred between the four main components during their mixing and processing of the film. Furthermore, the intensity mapping of HA NPs at 960 cm⁻¹ showed a good dispersion of the HA nanoparticles in the film, which confirms the observation of a uniform HA distribution obtained via SEM imaging.

Notwithstanding the limited resolution of the technique (> 1 □m), the INT-WS₂ were clearly seen as elongated shapes throughout the film in the Raman mapping. Obviously, the asymmetric shape of the nanotube does not reflect its genuine shape, since the coarse size of the focused laser beam (1-2 □m) is at least 10-times larger than the tube diameter (-100 nm). Moreover, the nanotubes are fully dispersed in the film. Their long axis seem to be within the film plan and oriented roughly in the x-direction. The preferred directionality of the tubes could be related to the mode of evaporation of the solvent from the casted film.

Raman mapping of oleic acid at 1655 cm⁻¹ presents relatively strong and uniform intensity throughout the film area, with minimum value of (normalized) intensity around 40%. Thus, it can be concluded that the oleic acid was uniformly dispersed throughout the nanocomposite film during its preparation.

### FTIR spectroscopy

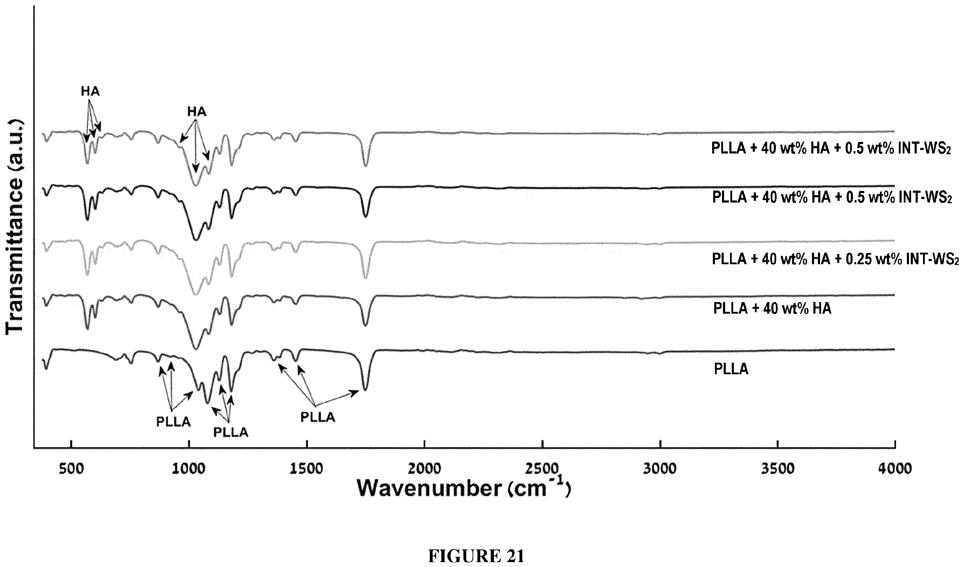

FTIR of different PLLA/HA/INT-WS₂ nanocomposite films was conducted and the results of the spectra are displayed in Figure 21.

Visibly, PLLA peaks were observed for all different films at the same position, except for the two peaks in 1044 cm⁻¹ and 1086 cm⁻¹ which overlap with the two IR peaks of HA in 1033 cm⁻¹ and 1093 cm⁻¹. No extra peaks occur due to the addition of HA and nanotubes to the PLLA.

The nanotubes peaks (<500 cm⁻¹) were not observed due to the predominant PLLA peak in this region. Thus, consistently with the previous measurements, the FTIR indicates that the four components (PLLA, HA, OA(=oleic acid) and INT) are mixed together uniformly and they are compatible with each.

## Claims

1. A composite comprising a hydrophobic biodegradable polymer, hydroxyapatite [Ca₁₀(PO₄)₆(OH)₂)] nanoparticles, a biocompatible surfactant and inorganic fullerene-like nanoparticles or inorganic nanotubes; wherein the inorganic fullerene-like nanoparticles or inorganic nanotubes is A₁₋ₓBₓ-chalcogenide where **A** is a metal or transition metal or an alloy of one metals or transition metals including at least one of the following: Mo, W, Re, Ti, Zr, Hf, Nb, Ta, Pt, Ru, Rh, In, Ga, InS, InSe, GaS, GaSe, WMo, TiW; and **B** (dopant) is a metal transition metal selected from the following: Si, Nb, Ta, W, Mo, Sc, Y, La, Hf, Ir, Mn, Ru, Re, Os, V, Au, Rh, Pd, Cr, Co, Fe, Ni; x is between 0 to 0.003; the chalcogenide is selected from S, Se, Te; and the biocompatible surfactant is a fatty acid having between 12-24 carbons.

2. The composite of claim 1, wherein the biodegradable polymer is poly(lactic acid) (PLA), Poly(L-lactide) (PLLA) or poly-D-lactide (PDLA).

3. The composite according to claim 1 or 2 wherein the biocompatible surfactant is oleic acid.

4. The composite according to any of the preceding claims, wherein the inorganic fullerene-like nanoparticles or inorganic nanotubes are WS₂, MoSz or combination thereof.

5. The composite according to any of the preceding claims, wherein the concentration of inorganic fullerene-like nanoparticles or inorganic nanotubes is between 0.1 wt% to 5 wt% of the composite.

6. The composite according to any of the preceding claims, wherein the concentration of HA is between 20 wt% to 60 wt% of the composite.

7. The composite according to any of the preceding claims, wherein the HA nanoparticles and the inorganic fullerene-like nanoparticles or inorganic nanotubes are dispersed in the PLLA.

8. The composite according to any of the preceding claims, wherein the composite is deposited on a substrate to form a film.

9. The composite of claim 8, wherein the film provides an improved hardness by 1.5 to 3, Young Modules by 1.5 to 3, Toughness by 2-10, Yield Strength by 1.2 to 3 and/or Strain at failure by 1.1 to 3 compared to PLLA film.

10. The composite of claim 8 or 9, wherein the film provides higher thermal stability compared to a PLLA film or a film comprising PLLA and HA.

11. The composite according to any of claims 8-10, wherein there is no chemical bonding between each of the PLLA, HA and the inorganic fullerene-like nanoparticles or inorganic nanotubes.

12. A method of coating of a substrate by a film, wherein the film comprises the composite according to any of claims 1-7, wherein the method comprises solvent casting.

13. The method of claim 12, wherein the metal substrate is titanium, alloys of titanium, Co-Cr alloys, magnesium, stainless steel, shape memory alloys of nickel-titanium, silver, tantalum, zirconium and novel ceramics or any electrical-conductive substrate.

14. An implant, a repaired bone or an engineered tissue comprising the composite according to any of claims 1-11.

## Patentansprüche

1. Komposit, umfassend ein hydrophobes biologisch abbaubares Polymer, Hydroxyapatit-[Ca₁₀(PO₄)₆(OH)₂)]-Nanopartikel, einen biologisch kompatiblen oberflächenaktiven Stoff und anorganische Fulleren-artige Nanopartikel oder anorganische Nanoröhren; wobei die anorganischen Fulleren-artigen Nanopartikel oder anorganischen Nanoröhren A₁₋ₓBₓ-Chalkogenid sind, wobei **A** ein Metall oder Übergangsmetall oder eine Legierung aus Metallen oder Übergangsmetallen ist, einschließlich mindestens einem der folgenden: Mo, W, Re, Ti, Zr, Hf, Nb, Ta, Pt, Ru, Rh, In, Ga, InS, InSe, GaS, GaSe, WMo, TiW; und wobei **B** (Dotiermittel) ein Metall oder Übergangsmetall ist, das unter den folgenden ausgewählt ist: Si, Nb, Ta, W, Mo, Sc, Y, La, Hf, Ir, Mn, Ru, Re, Os, V, Au, Rh, Pd, Cr, Co, Fe, Ni; wobei x zwischen 0 und 0,003 ist; wobei das Chalkogenid unter S, Se, Te ausgewählt ist; und wobei der biologisch abbaubare oberflächenaktive Stoff eine Fettsäure mit 12-24 Kohlenstoffatomen ist.

2. Komposit nach Anspruch 1, wobei das biologisch abbaubare Polymer Poly(milchsäure) (PLA), Poly(L-Lactid) (PLLA) oder Poly-D-Lactid (PDLA) ist.

3. Komposit nach Anspruch 1 oder 2, wobei der biologisch abbaubare oberflächenaktive Stoff Oleinsäure ist.

4. Komposit nach einem der vorhergehenden Ansprüche, wobei die anorganischen Fulleren-artigen Nanopartikel oder anorganischen Nanoröhren WS₂, MoS₂ oder Kombinationen davon sind.

5. Komposit nach einem der vorhergehenden Ansprüche, wobei die Konzentration von anorganischen Fulleren-artigen Nanopartikeln oder anorganischen Nanoröhren zwischen 0,1 Gew% und 5 Gew% des Komposits ist.

6. Komposit nach einem der vorhergehenden Ansprüche, wobei die Konzentration von HA zwischen 20 Gew% und 60 Gew% des Komposites ist.

7. Komposit nach einem der vorhergehenden Ansprüche, wobei die HA-Nanopartikel und die anorganischen Fulleren-artigen Nanopartikel oder anorganischen Nanoröhren in PLLA dispergiert sind.

8. Komposit nach einem der vorhergehenden Ansprüche, wobei das Komposit auf ein Substrat aufgebracht wird, um einem Film zu bilden.

9. Komposit nach Anspruch 8, wobei der Film verglichen mit einem PLLA-Film eine um 1,5 bis 3 verbesserte Härte, einen um 1,5 bis 3 verbesserten Young modulus, eine um 2-10 verbesserte Schlagzähigkeit, eine um 1,2 bis 3 verbesserte Streckfestigkeit und/oder eine um 1,1 bis 3 verbesserte Bruchdehnung bereitstellt.

10. Komposit nach Anspruch 8 oder 9, wobei der Film verglichen mit einem PLLA-Film oder einem Film, der PLLA und HA umfasst, eine verbesserte Wärmebeständigkeit bereitstellt.

11. Komposit nach einem der Ansprüche 8-10, wobei zwischen jedem von PLLA, HA und den anorganischen Fulleren-artigen Nanopartikeln oder anorganischen Nanoröhren keine chemische Bindung besteht.

12. Verfahren zum Beschichten eines Substrats mit einem Film, wobei der Film das Komposit nach einem der Ansprüche 1-7 umfasst, wobei das Verfahren einen Lösungsmittelguss umfasst.

13. Verfahren nach Anspruch 12, wobei das Metallsubstrat aus Titan, Titanlegierungen, Co-Cr-Legierungen, Magnesium, Edelstahl, Formgedächtnislegierungen aus Nickel-Titan, Silber, Tantal, Zirconium und neuartigen Keramikstoffen ist oder wobei es ein beliebiges elektrisch leitendes Substrat ist.

14. Implantat, ein reparierter Knochen oder ein künstlich hergestelltes Gewebe, umfassend das Komposit nach einem der Ansprüche 1-11.

## Revendications

1. Composite comprenant un polymère biodégradable hydrophobe, des nanoparticules d'hydroxyapatite [Ca₁₀(PO₄)₆(OH)₂)], un tensioactif biocompatible et des nanoparticules inorganiques de type fullerène ou des nanotubes inorganiques ; dans lequel les nanoparticules inorganiques de type fullerène ou les nanotubes inorganiques sont le A1-ₓ Bₓ-chalcogénure où **A** est un métal ou un métal de transition ou un alliage d'un métal ou de métaux de transition comprenant au moins l'un des éléments suivants : Mo, W, Re, Ti, Zr, Hf, Nb, Ta, Pt, Ru, Rh, In, Ga, InS, InSe, GaS, GaSe, WMo, TiW ; et **B** (dopant) est un métal de transition choisi parmi les éléments suivants : Si, Nb, Ta, W, Mo, Sc, Y, La, Hf, Ir, Mn, Ru, Re, Os, V, Au, Rh, Pd, Cr, Co, Fe, Ni ; x est compris entre 0 et 0,003 ; le chalcogénure est choisi parmi S, Se, Te ; et le tensioactif biocompatible est un acide gras ayant entre 12 et 24 carbones.

2. Composite selon la revendication 1, dans lequel le polymère biodégradable est le poly(acide lactique) (PLA), le Poly(L-lactide) (PLLA) ou le poly-D-lactide (PDLA).

3. Composite selon la revendication 1 ou 2, dans lequel le tensioactif biocompatible est l'acide oléique.

4. Composite selon l'une quelconque des revendications précédentes, dans lequel les nanoparticules inorganiques de type fullerène ou les nanotubes inorganiques sont du WS₂, du MoS₂ ou une combinaison de ceux-ci.

5. Composite selon l'une quelconque des revendications précédentes, dans lequel la concentration de nanoparticules inorganiques de type fullerène ou de nanotubes inorganiques est comprise entre 0,1 % en poids et 5 % en poids du composite.

6. Composite selon l'une quelconque des revendications précédentes, dans lequel la concentration de HA est comprise entre 20 % en poids et 60 % en poids du composite.

7. Composite selon l'une quelconque des revendications précédentes, dans lequel les nanoparticules de HA et les nanoparticules inorganiques de type fullerène ou les nanotubes inorganiques sont dispersés dans le PLLA.

8. Composite selon l'une quelconque des revendications précédentes, dans lequel le composite est déposé sur un substrat pour former un film.

9. Composite selon la revendication 8, dans lequel le film offre une dureté améliorée de 1,5 à 3, des modules de Young de 1,5 à 3, une résistance de 2 à 10, une limite d'élasticité de 1,2 à 3 et/ou une déformation à la rupture de 1,1 à 3 par rapport au film PLLA.

10. Composite selon la revendication 8 ou 9, dans lequel le film offre une stabilité thermique supérieure par rapport à un film PLLA ou à un film comprenant du PLLA et du HA.

11. Composite selon l'une quelconque des revendications 8 à 10, dans lequel il n'y a aucune liaison chimique entre chacun parmi le PLLA, le HA et les nanoparticules inorganiques de type fullerène ou les nanotubes inorganiques.

12. Procédé de revêtement d'un substrat par un film, dans lequel le film comprend le composite selon l'une quelconque des revendications 1 à 7, le procédé comprenant un coulage au solvant.

13. Procédé selon la revendication 12, dans lequel le substrat métallique est du titane, des alliages de titane, des alliages Co-Cr, du magnésium, de l'acier inoxydable, des alliages de nickel-titane à mémoire de forme, de l'argent, du tantale, du zirconium et de nouvelles céramiques ou tout substrat conducteur d'électricité.

14. Implant, os réparé ou tissulaire modifié comprenant le composite selon l'une quelconque des revendications 1 à 11.
